(19) Europäisches Patentamt
European Patent Office
Office européen des brevets

(11) EP 0 576 949 B1

(12) **EUROPÄISCHE PATENTSCHRIFT**

(45) Veröffentlichungstag und Bekanntmachung des Hinweises auf die Patenterteilung:
**31.01.1996 Patentblatt 1996/05**

(51) Int. Cl.$^6$: **C07C 317/50**, C07D 277/06, C08F 20/54, C07B 57/00

(21) Anmeldenummer: **93109872.7**

(22) Anmeldetag: **21.06.1993**

(54) **Optisch aktive Aminosäuresulfoxid- und -sulfon-Derivate, ihre Herstellung, ihre Polymerisation und Verwendung als Adsorbentien für die chromatographische Racematspaltung**

Optically active amino acid sulphoxide and sulphone derivatives, their preparation, their polymerisation and use as adsorbents for chromatograpic separation of racemates

Dérivés des sulfoxydes et des sulfones des aminoacides optiquement actifs, leur polymérisation et leur utilisation comme des adsorbants pour la séparation chromatographique des racémates

(84) Benannte Vertragsstaaten:
**AT BE CH DE DK ES FR GB GR IE IT LI LU MC NL PT SE**

(30) Priorität: **02.07.1992 DE 4221711**

(43) Veröffentlichungstag der Anmeldung:
**05.01.1994 Patentblatt 1994/01**

(73) Patentinhaber: **BAYER AG**
**D-51368 Leverkusen (DE)**

(72) Erfinder:
- **Grosse-Bley, Michael, Dr.**
  **D-51061 Köln (DE)**
- **Bömer, Bruno, Dr.**
  **D-51467 Bergisch Gladbach (DE)**
- **Grosser, Rolf, Dr.**
  **D-51373 Leverkusen (DE)**
- **Lange, Walter, Dr.**
  **D-50733 Köln (DE)**
- **Hoever, Franz-Peter, Dr.**
  **D-51069 Köln (DE)**
- **Arlt, Dieter, Prof. Dr.**
  **D-51065 Köln (DE)**

(56) Entgegenhaltungen:
**EP-A- 0 379 917**
**EP-A- 0 464 488**
**EP-A- 0 520 242**
**US-A- 3 846 306**
**US-A- 3 884 761**

EP 0 576 949 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.9.8/3.4

## Beschreibung

Die Erfindung betrifft neue optisch aktive Aminosäurederivate, die Sulfoxid- bzw. Sulfongruppen enthalten, zwei grundsätzliche Verfahrenswege zu deren Herstellung aus schwefelhaltigen Aminosäuren, die Polymerisation dieser Monomeren und die Verwendung der Polymeren als optisch aktive Adsorbentien für die chromatographische Trennung von Racematen in die Enantiomere.

Die Auftrennung biologisch wirksamer Racemate in die Enantiomere hat in den letzten Jahren eine immer größere Bedeutung erlangt, weil sich herausstellte, daß sich die Enantiomere eines Racemates in der Regel stark in ihren Wirkungen und Nebenwirkungen unterscheiden. Daher besteht meist schon im sehr frühen Entwicklungsstadium z.B. einer neuen Pharmasubstanz von Seiten der Pharmakologie oder der Toxikologie ein großes Interesse am Vorliegen ausreichender Mengen beider Enantiomere eines chiralen Wirkstoffes. Über die chromatographische Racematspaltung können oft leicht und kurzfristig hinreichende Mengen beider Enantiomere für die weiteren biologischen Prüfungen (immer mehr auch im Pflanzenschutz) zu einem frühen Zeitpunkt, zu dem häufig eine enantioselektive Synthese noch nicht vorliegt und die klassische Racematspaltung über diastereomere Salze nur in schlechten Ausbeuten verläuft, zur Verfügung gestellt werden.

Nun wurden bereits die verschiedensten Adsorbentien für die chromatographische Racematspaltung vorgeschlagen. Als brauchbare Adsorptionsmittel haben sich dabei bislang z.B. die in EP-A 379 917 beschriebenen polymeren (Meth)Acrylsäure-Derivate optisch aktiver Amino-Verbindungen erwiesen, gegebenenfalls in an anorganischen Trägern immobilisierter Form.

Es wurde nun überraschend gefunden, daß optisch aktive Sulfoxide und Sulfone (im Folgenden S-Oxide genannt) von schwefelhaltigen Aminosäurederivaten, die eine polymerisierbare Gruppe enthalten, zu sehr interessanten neuen optisch aktiven chromatographischen Adsorbentien führen, die bei einer Reihe von Racematen deutlich verbesserte Trennergebnisse im Vergleich zu bekannten Trennmaterialien, besonders schwefelfreien bzw. S-Oxid-freien Aminosäurederivaten, liefern.

Die Erfindung betrifft daher optisch aktive S-Oxide enthaltende Aminosäurederivate der Formel (I)

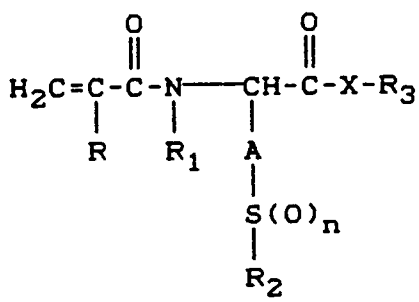

(I),

in der

n den wert 1 oder 2 hat,

R für Wasserstoff, Methyl oder Fluor steht,

$R_1$ für Wasserstoff oder $C_1$-$C_4$-Alkyl steht oder zusammen mit $R_2$ eine Methylengruppe oder eine Dimethylengruppe bildet, die ein- oder zweifach mit $C_1$-$C_4$-Alkyl substituiert sein können,

$R_2$ für einen geradkettigen, verzweigten oder cyclischen Alkylrest mit bis zu 10 C-Atomen, $C_6$-$C_{14}$-Aryl,

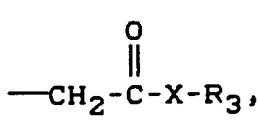

$C_2$-$C_{10}$-Acyl, gegebenenfalls substituiertes Benzoyl- oder Benzyl steht oder zusammen mit $R_1$ eine dort beschriebene Brücke bildet,

$R_3$ für einen geradkettigen, verzweigten oder cyclischen Alkylrest mit bis zu 20 C-Atomen steht, der gegebenenfalls ein- bis dreifach substituiert ist durch Halogen, Alkoxy mit 1 bis 4 C-Atomen, Aralkoxy mit 7 bis 16 C-Atomen oder Aryl mit 6 bis 10 C-Atomen,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der ein- oder zweifach $C_1$-$C_4$-alkyl- oder $C_1$-$C_6$-alkoxycarbonylsubstituiert sein kann,

A für eine gegebenenfalls ein- oder zweifach $C_1$-$C_4$-alkylsubstituierte Methylen- oder Dimethylen-Gruppe steht.

Bevorzugte Verbindungen der Formel (I) sind solche, in denen

n den Wert 1 oder 2 hat,

R für Wasserstoff, Methyl oder Fluor steht,

$R_1$ Wasserstoff oder Methyl bedeutet oder zusammen mit $R_2$ eine Methylengruppe, die ein- oder zweifach Methyl- oder einfach tertiär-Butyl-substituiert sein kann, oder eine Dimethylengruppe bildet,

$R_2$ für Alkyl mit bis zu 8 C-Atomen, Phenyl, Benzyl, Benzoyl und

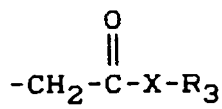

steht
oder zusammen mit $R_1$ eine dort beschriebene Brücke bildet,

$R_3$ für einen $C_{10}$-Terpenylrest, einen Adamantylrest, einen Decahydronaphthylrest, einen 1-Phenylethyl-Rest, einen $C_5$-$C_{12}$-Cycloalkylrest wie Cyclopentyl-, Cyclohexyl- oder Cycloheptyl- oder einen verzweigten $C_3$-$C_{12}$-Alkylrest wie Isopropyl, 3-Pentyl oder 5-Heptyl steht,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der gegebenenfalls ein-oder zweifach $C_1$-$C_4$-alkyl- oder $C_1$-$C_6$-alkoxycarbonyl-substituiert sein kann,

A für eine Methylen-, Dimethylmethylen- oder eine Dimethyleneinheit steht.

Die optisch aktiven Aminosäure-Derivate leiten sich vorzugsweise von optisch aktiven schwefelhaltigen Aminosäuren wie z.B. Cystein, Penicillamin oder Homocystein ab. Von besonderem Interesse sind dafür Verbindungen, deren SH-Funktion alkyliert ist wie z.B. in S-Methylcystein, S-Benzyl-cystein, S-Methyl-penicillamin, Methionin, Ethionin oder Butionin, oder acyliert ist wie z.B. in S- Acetyl-cystein, oder Alkoxycarbonylmethyliert ist wie z.B. in S-Menthoxycarbonylmethyl-cystein, oder über eine Alkylenbrücke mit der Aminogruppe verbunden ist wie z.B. in Thioprolin, 2,2-Dimethyl-thioprolin, 5,5-Dimethyl- thioprolin oder 2-tert.-Butylthioprolin.

Für $R_3$ ist die Verwendung optisch aktiver Reste besonders vorteilhaft, z.B. des 1-Phenylethyl-, 1-(1-Naphthylethyl)-, l-(2-Naphthylethyl)-, des d- oder l-Menthyl, d- oder l-Neomenthyl-, d- oder l-Bornyl-, d-oder l-Fenchyl-, des d- oder l-Pinanylrestes oder des 3-Methylenylpinanyl-Restes.

Die erfindungsgemäßen optisch aktiven S-Oxide enthaltenden Aminosäurederivate der Formel (I) sind herstellbar durch Umsetzung von optisch aktiven Aminosäure-S-Oxiden der Formel (II)

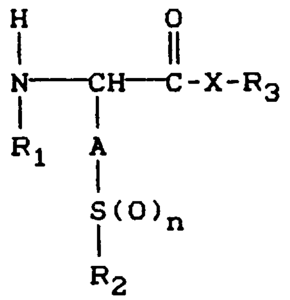

(II),

in der
n, $R_1$, $R_2$, $R_3$, A und X die unter Formel (I) angegebene Bedeutung haben,
oder deren Säureadditionsprodukten mit Acryloyl-Derivaten der Formel (III)

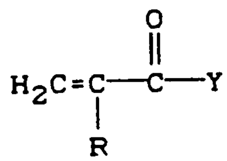

(III),

in der

R die unter Formel (I) angegebene Bedeutung hat und

Y für Fluor, Chlor oder Brom steht oder für den Rest

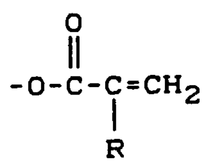

steht (Anhydrid),

gegebenenfalls in Gegenwart eines Säurebindemittels in inerten organischen Lösungsmitteln.

Geeignete Säureadditionsverbindungen der als Ausgangsstoffe zu verwendenden Aminosäure-S-Oxide sind Salze dieser Aminosäure-S-Oxide mit anorganischen oder organischen Säuren. Bevorzugt sind Mineralsäuren wie Salzsäure, Bromwasserstoffsäure, Schwefelsäure, Phosphorsäure oder organische Säuren wie Essigsäure, Trifluoressigsäure, Methan-, Ethan-, Benzol- oder Toluolsulfonsäure.

Als Lösungsmittel eignen sich alle unter den Reaktionsbedingungen inerten organischen Lösungsmittel. Bevorzugt sind Kohlenwasserstoffe wie Toluol, Petrolether oder Halogenkohlenwasserstoffe wie Dichlormethan, Dichlorethan oder Trichlorethylen oder Ether wie tert.-Butylmethylether.

Als Säurebindemittel kommen vor allem die üblichen anorganischen oder organischen Basen in Betracht; bevorzugt werden Alkali- oder Erdalkalihydroxide wie Natrium-, Kalium-, Lithium-, Calcium- oder Bariumhydroxid, Alkali-oder Erdalkalicarbonate wie Natrium- oder Kaliumcarbonat oder Natriumbicarbonat, Alkalialkoholate wie Natriummethylat, -ethylat, Kaliummethylat, -ethylat, -tert.-butylat oder Amine wie Triethylamin oder Pyridin verwendet.

Die Umsetzung der Acryloyl-Derivate der Formel (III) mit den S-Oxiden der Formel (II) wird bevorzugt bei Temperaturen von -78 bis +100°C, insbesondere von -20 bis +60°C vorgenommen.

Die als Ausgangsverbindungen verwendeten optisch aktiven Aminosäure-S-oxide der Formel (II) können nach an sich bekannten Verfahren dargestellt werden.

Will man im Falle der Sulfoxide (n=1 in Formel (II)) zu diastereo- und enantiomerenreinen Verbindungen der Formel (II) gelangen, wird die Oxidation des Schwefels günstigerweise auf der Stufe der freien, schwefelhaltigen Aminosäure z.B. mit Wasserstoffperoxid durchgeführt. Das dann entstehende Diastereomerengemisch (neues asymmetrisches Zentrum am Schwefel) kann durch die unterschiedliche Löslichkeit diastereomerer Salze aufgetrennt werden.

Die Oxidation der freien Aminosäure L-Methionin zum Sulfoxid und die anschließende Trennung des 1:1-Diastereomerengemisches über die unterschiedliche Löslichkeit der Pikrate in Wasser ist bekannt. Aus dem schwerlöslichen Pikrat läßt sich das am Schwefel (S)-konfigurierte Diastereomer nach Freisetzen mit n-Pentylamin erhalten, aus dem löslichen das am Schwefel (R)-konfigurierte.

Der weitere Weg verläuft über bekannte Verfahren der Peptidchemie wie Einführung einer N-Schutzgruppe (z.B. tert.-Butoxycarbonyl), Darstellung des Esters bzw. Amides durch Kupplung mit den entsprechenden Alkoholen bzw. Aminen (Dicyclohexylcarbodiimid-Methode bzw. Aktivierung durch gemischtes Anhydrid mit Chlorameisensäureester) und Abspaltung der N-Schutzgruppe zu Verbindungen der Formel (II).

Auch die Sulfone (n=2 in Formel (II)) lassen sich grundsätzlich auf dem oben beschriebenen Verfahrensweg darstellen. Die Diastereomerentrennung zu Beginn entfällt hier natürlich.

In einem zweiten alternativen Verfahrensweg wird die Oxidation des Schwefels erst im letzten Schritt durchgeführt. Dieses Verfahren eignet sich besonders für Sulfone (n=2 in Formel (I)) und 1:1-Diastereomerengemische von Sulfoxiden (n=1 in Formel (I)). Die erfindungsgemäßen optisch aktiven S-Oxide enthaltenden Aminosäurederivate der Formel (I) werden dann erhalten durch Oxidation von optisch aktiven schwefelhaltigen Aminosäurederivaten der Formel (IV)

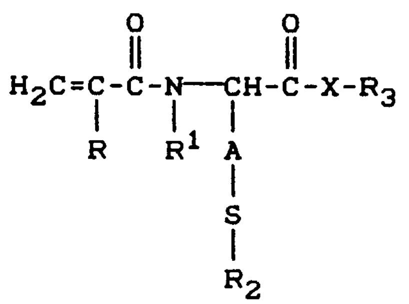

(IV),

in der

R, $R_1$, $R_2$, $R_3$, A und X die unter Formel (I) angegebene Bedeutung haben,

mit Oxidationsmitteln wie Wasserstoffperoxid, Natriummetaperiodat, 3-Chlorperbenzoesäure, Magnesiummonoperoxyphthalsäure, Tripelsalze der Caro'schen Säure ($KHSO_5xKHSO_4xK_2SO_4$) und anderen Sauerstoffüberträgern, die geeignet sind, Thioether in Sulfoxide und Sulfone zu überführen.

Als Lösungsmittel eignen sich besonders Alkohole wie Methanol und Ethanol, Essigsäure, Wasser und Methylenchlorid und Gemische dieser Lösungsmittel.

Die Oxidation der schwefelhaltigen Aminosäurederivate der Formel (IV) wird bevorzugt bei Temperaturen von -78°C bis +100°C, besonders bevorzugt von -20°C bis +60°C durchgeführt.

Es werden bevorzugt 1 bis 1,5 Äquivalente Oxidationsmittel zur Darstellung der Sulfoxide (n=1, Formel (I)) bzw. 2 bis 3 Äquivalente Oxidationsmittel zur Darstellung der Sulfone (n=2, Formel (I)) jeweils aus den schwefelhaltigen Aminosäurederivaten der Formel (IV) angewendet.

Die als Ausgangsverbindungen verwendeten optisch aktiven schwefelhaltigen Aminosäurederivate der Formel (IV) können nach an sich bekannten Verfahren der Peptidchemie dargestellt werden (vgl. Weg zu (I) über (II), aber jeweils n=0).

Ein um die Schutzgruppenchemie verkürztes Verfahren zu Ausgangsstoffen der Formel (IV) benutzt die direkte Umsetzung der freien Aminosäure im Basischen mit den entsprechenden Acryloyl-Derivaten der Formel (III) und die Kupplung der N-acryloylierten Aminosäuren mit den entsprechenden Aminen bzw. Alkoholen ($R_3$-X-H) unter Verwendung von 1,2-Dihydro-2-ethoxychinolin-1-carbonsäureethylester (EEDQ-Methode) bzw. N,N'-Dicyclohexylcarbodiimid (DCC-Methode).

Die Erfindung betrifft auch die durch Polymerisation bzw. Copolymerisation der optisch aktiven Acryloylaminosäure-S-Oxid-Derivate der Formel (I) erhältlichen optisch aktiven Polymere und Copolymere, die mindestens 40 Mol-%, vorzugsweise mindestens 50 Mol-% Struktureinheiten der Formel (V)

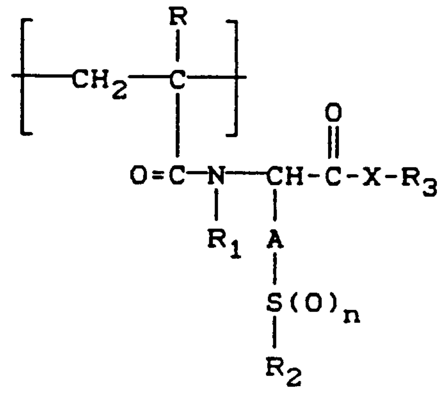

(V)

enthalten, in der

n, R, $R_1$, $R_2$, $R_3$, A und X die unter Formel (I) angegebene Bedeutung haben.

Vorzugsweise liegen die erfindungsgemäßen optisch aktiven Polymere der Formel (V) in Form von vernetzten unlöslichen aber quellbaren Perlpolymerisaten oder in an feinteilige anorganische Trägermaterialien wie z.B. Kieselgel gebundener Form vor, Sie können auch als lineare, in geeigneten organischen Lösungsmitteln lösliche Polymere hergestellt werden, Es ist ferner möglich, verschiedene erfindungsgemäße schwefelhaltige Acryloylaminosäure-S-Oxid-Derivate der Formel (I) cozupolymerisieren, sowie 0,1 bis 60, vorzugsweise 0,1 bis 20 Mol-% copolymerisierbarer, anderer Monomere in die Polymere einzubauen.

Die vernetzten Polymerisate liegen vorzugsweise in Form kleiner Teilchen (Perlen) mit 5 bis 200 µm Teilchendurchmesser vor. Sie werden z.B. durch Suspensionspolymerisation der optisch aktiven schwefelhaltigen Acryloylaminosäure-S-Oxid-Derivate der Formel (I) mit 0,5 bis 50 Mol-%, vorzugsweise 1 bis 30 Mol-%, besonders bevorzugt 3 bis 20 Mol-% (bezogen auf die Gesamtmenge [Mol] der eingesetzten Monomere) eines geeigneten Vernetzers in an sich bekannter Weise hergestellt.

Durch die Art und Menge der Vernetzer und des Lösungsmittels läßt sich der Quellungsgrad der (Perl)Polymerisate nach üblichen Methoden einstellen.

Bei der praktischen Verwendung haben sich (Perl)Polymerisate mit einem Quellungsgrad (Q) von 1.1 bis 12.0, bevorzugt von 2,0 bis 6.0 bewährt.

Der Quellungsgrad Q wird wie folgt bestimmt:

$$Q = \frac{\text{Polymerisationsvolumen (gequollen)}}{\text{Polymerisationsvolumen (ungequollen)}}$$

Als Vernetzungsmittel kommen Verbindungen in Frage, die mindestens zwei polymerisierbare Vinylgruppen enthalten. Bevorzugte Vernetzungsmittel sind Alkandioldiacrylate wie 1,6-Hexandioldiacrylat, 1,4-Butandioldiacrylat, 1,3-Propandioldiacrylat oder 1,2-Ethylenglykoldiacrylat, oder Alkandioldimethacrylate wie 1,4-, 1,3- oder 2,3-Butandioldimethacrylat, 1,3-Propandioldimethacrylat oder 1,2-Ethylenglykoldimethacrylat, aromatische Divinylverbindungen wie beispielsweise Divinylbenzol, Divinylchlorbenzol oder Divinyltoluol, Dicarbonsäuredivinylester wie Adipinsäuredivinylester, Benzoldicarbonsäuredivinylester, Terephthalsäuredivinylester, N,N'-Alkylendiacrylamide wie N,N'-Methylendiacrylamid, N,N'-Ethylendiacrylamid, N,N'-Methylendimethacrylamid, N,N'-Ethylendimethacrylamid oder N,N'-Dimethylethylendiacrylamid. Ebenso kann N,N',N''-Tris(Acryloyl)-1,3,5-perhydrotriazin oder N,N'-Bis(acryloyl)piperazin verwendet werden.

Als Radikalbildner kommen die üblichen Radikalbildner in Frage. Bevorzugt sind Peroxide wie beispielsweise Dibenzoylperoxid, Dilauroylperoxid oder Di-orthotoluoylperoxid, Perester wie tert.-Butylperpivalat oder tert.-Butylperoctanoat, oder Azoverbindungen wie beispielsweise Azobisisobuttersäurenitril (AIBN). Auch Gemische verschiedener Radikalbildner sind verwendbar.

Die Polymerisationskomponenten werden in einem nicht mit Wasser mischbaren organischen Lösungsmittel, vorzugsweise einem aliphatischen oder aromatischen Kohlenwasserstoff wie Hexan, Heptan, Isodecan, Benzol oder Toluol, einem Halogenkohlenwasserstoff wie Di-, Tri-, Tetrachlormethan oder 1,2-Dichlorethan, einem Ester wie Essigsäureethylester, Essigsäurebutylester oder Dialkylcarbonaten oder einem nicht wasserlöslichen Keton wie Methylisobutylketon oder Cyclohexanon gelöst.

Die organische Phase wird mit Hilfe eines wirksamen Rührers in der wäßrigen Lösung eines Schutzkolloids, bevorzugt in einer wäßrigen Lösung von Polyvinylalkohol, Polyvinylpyrrolidon oder eines Copolymers aus Methacrylsäure und Methylmethacrylat, gleichmäßig verteilt. Je Gewichtsteil organische Phase verwendet man etwa 1 bis 20, bevorzugt

2 bis 10 Gew.-Teile wäßrige Phase. Das Polymerisationsgemisch wird unter Rühren in einer Inertgasatmosphäre, vorzugsweise unter Stickstoff, auf Temperaturen von 30°C bis 100°C, vorzugsweise 40°C bis 80°C erhitzt. Die Polymerisationsdauer beträgt zwischen 2 und 24, bevorzugt 4 und 12 Stunden, Das auf diese Weise erhaltene Copolymerisat wird durch Filtration von der flüssigen Phase getrennt, durch gründliches Waschen mit Wasser und mit organischen Lösungsmitteln wie Methanol, Ethanol, Benzol, Toluol, Di-, Trichlormethan oder Aceton gereinigt und anschließend getrocknet.

Insbesondere für analytische Anwendungen werden die erfindungsgemäßen optisch aktiven Polymere vorzugsweise in an feinteilige anorganische Träger gebundener Form eingesetzt. Die Herstellung solcher optisch aktiver Chromatographiephasen kann beispielsweise nach den in DE-A 3 706 890 beschriebenen Verfahren erfolgen.

Bevorzugt ist die Polymerisation der optisch aktiven schwefelhaltigen Aminosäure-S-Oxid-Derivate der Formel (I) in Gegenwart von Kieselgel-Vinylphasen, die nach bekannten Methoden erhältlich sind, oder von Kieselgel-Diolphasen, die mit (Meth)acrylsäure verestert wurden. Die Polymerisation kann dabei in Abwesenheit von Lösungsmitteln oder in Gegenwart von Lösungsmitteln oder von Fällungsmitteln für die Poly-N-Acryloylamid-S-Oxid-Derivate durchgeführt werden, Als Initiatoren können die zur Herstellung der Perlpolymerisate verwendeten Radikalbildner ebenfalls eingesetzt werden.

Die polymermodifizierten Kieselgele enthalten vorzugsweise 1 bis 40 Gew.-%, insbesondere 5 bis 30 Gew.-% an optisch aktivem Monomer (I), bezogen auf das Gesamtgewicht, Sie werden intensiv mit Lösungsmitteln für das Polymere gewaschen und im Vakuum getrocknet.

Selbstverständlich können auch hier Gemische von zwei oder mehreren der erfindungsgemäßen N-Acryloylaminosäure-S-Oxid-Derivate, gegebenenfalls auch mit weiteren copolymerisierbaren Monomeren eingesetzt werden.

Die Erfindung betrifft ferner die Verwendung der erfindungsgemäßen Polyacrylamid-S-oxide als solche oder in vernetzter bzw. an Kieselgel gebundener Form zur chromatographischen Trennung von racemischen Gemischen in die optischen Antipoden. Besonders bewährt haben sich die erfindungsgemäßen Polymerisate zur chromatographischen Trennung von Hexahydrocarbazol-Derivaten wie z.B. 3-r-(4-Fluorphenylsulfonamido)-9-(2-carboxyethyl)-1,2,3,4,4a,9a-hexahydrocarbazol, Benzodiazepinen wie Oxazepam, Arylpropionsäureamide wie Ketoprofen- und Ibuprofenamid, Dihydropyridinen wie z.B. 1,4-Dihydro-2,6-dimethyl-4-(2-trifluormethylphenyl)pyridin-3,5-dicarbonsäure-5-methylester, Binaphthol, Benzoin und Hydrobenzoin, Stilbenoxid, Chlorthalidon, Thiaprofen und Mandelamid.

Die Zusammensetzung des Fließmittels kann je nach Art und Eigenschaft des zu trennenden Racemates in üblicher Weise ausgewählt und optimiert werden. Die im Kieselgel gebundenen erfindungsgemäßen Polyacrylamid-S-oxide können für chromatographische Racemattrennungen unter HPLC-Bedingungen eingesetzt werden.

Die Fähigkeit der Polymerisate zur Racemattrennung wird durch die Kapazitätsverhältnisse ($k'_{1(2)}$-Werte) für die beiden Enantiomere (1) und (2) und den daraus resultierenden Enantioselektivitätswert $\alpha$ ausgedrückt. Diese chromatographischen Parameter sind wie folgt definiert:

$$\text{Kapazitätsverhältnis } k'_{1(2)} = \frac{t_{1(2)} - t_o}{t_o}$$

$$\text{Enantioselektivität } \alpha = \frac{k'_2}{k'_1}$$

$t_o$ = Totzeit der Säule

$t_{1(2)}$ = Retentionszeit des zuerst eluierten Enantiomers 1 bzw. des später eluierten Enantiomers 2

Die präparative Trennung von racemischen Gemischen in ihre optischen Antipoden unter Verwendung der erfindungsgemäßen Polymerisate wird vorzugsweise säulenchromatographisch vorgenommen. Besonders vorteilhaft ist es hierfür, die chromatographische Trennung mit Perlpolymerisaten einer bestimmten Korngrößenverteilung vorzunehmen; gute Trennleistungen werden mit Perlpolymerisaten einer Korngrößenverteilung von 5 bis 200 µm, bevorzugt 15 bis 100 µm erhalten.

Die Arbeitsmethodik der säulenchromatographischen Trennung ist bekannt. Üblicherweise wird das Polymerisat in Fließmittel suspendiert und die Suspension in eine Glassäule gefüllt. Nach Ablaufen des Fließmittels wird das zu trennende Racemat, gelöst im Fließmittel, auf die Säule aufgebracht. Dann wird mit Fließmittel eluiert und die Enantiomeren im Eluat photometrisch und/oder polarimetrisch mittels geeigneter Durchflußzellen detektiert.

Als Fließmittel werden üblicherweise organische Lösungsmittel bzw. Lösungsmittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, tert.-Butylmethylether, Dioxan oder Tetrahydrofuran, Halogenkohlenwasserstoffe wie Di- oder Trichlormethan, Aceton, Acetonitril oder Essigester, Alkohole wie

Methanol, Ethanol, n-Propanol, Isopropanol oder n-Butanol, oder aber Gemische der genannten Lösungsmittel. Als besonders geeignet haben sich Mischungen aus Toluol mit Tetrahydrofuran, Dioxan oder Isopropanol erwiesen.

Beispiele

1) Darstellung der optisch aktiven S-Oxide enthaltenden Aminosäurederivate der Formel (I) aus (II) und (III)

Beispiel 1

a) (S)-L-Methionin-sulfoxid und (R)-L-Methionin-sulfoxid
Darstellung und Trennung der diastereomeren Sulfoxide aus L-Methionin über die Pikrate nach J. Biol. Chem. 169 (1947) 477.

b) (S)-N-BOC-L-Methionin-sulfoxid
0,1 mol (S)-L-Methionin-sulfoxid in 300 ml Dioxan:Wasser = 2:1 werden bei 5°C mit 0,1 mol $NaHCO_3$ und 100 ml 1N Natronlauge versetzt. Man gibt 0,11 mol $(BOC)_2O$ zu, läßt auf RT kommen und rührt weitere 30 min nach. Das Dioxan wird am Rotationsverdampfer abgezogen, die wäßrige Phase unter Eiskühlung und Überschichten mit Essigester durch 4N $KHSO_4$-Lösung auf pH2-3 gebracht. Man sättigt mit Kochsalz und extrahiert noch zweimal mit Essigester. Man trocknet mit $MgSO_4$ und engt ein.
Ausbeute: 13,8 g = 52%
$[\alpha]_D$: +91,8° (c=1,$H_2O$).

c) (S)-N-BOC-L-methionin-l-menthylamid-sulfoxid
50 mmol N-BOC-(S)-L-methionin-sulfoxid werden bei 0°C in 125 ml abs. THF mit 50 mmol $NEt_3$ versetzt. Dann tropft man bei -12°C 50 mmol Chlorameisensäureethylester zu. Nach 15 min gibt man 50 mmol L-Menthylamin zu. Man läßt bei 0°C 30 min nachrühren und dann auf RT kommen. Man engt ein, nimmt in $CH_2Cl_2$ auf, wäscht mit 1N Salzsäure, trocknet über $MgSO_4$ und engt erneut ein.
Ausbeute: 20 g = 100%

d) (S)-L-Methionin-l-menthylamid-sulfoxid
50 mmol N-BOC-(S)-L-methionin-l-menthylamin-sulfoxid werden in 100 ml $CH_2Cl_2$ bei 0°C mit 100 ml frisch destillierter Trifluoressigsäure tropfenweise versetzt. Man läßt auf RT kommen und rührt 1 h nach. Man engt bei RT ein, nimmt erneut in 200 ml $CH_2Cl_2$ auf und bringt bei 0°C unter Rühren mit 15%iger NaOH auf pH 12. Die organische Phase wird rasch abgetrennt, die wäßrige noch zweimal mit $CH_2Cl_2$ extrahiert. Die vereinigten organischen Phasen werden einmal mit Wasser gewaschen, über $MgSO_4$ getrocknet und eingeengt.
Ausbeute: 15 g = 100%

e) (S)-N-Methacryloyl-L-methionin-l-menthylamid-sulfoxid
50 mmol (S)-L-Methionin-l-menthylamid-sulfoxid werden in 40 ml $CH_2Cl_2$ bei 0°C mit 50 mmol $NEt_3$ versetzt. Bei -10°C werden 50 mmol frisch destilliertes Methacryloylchlorid in 30 ml $CH_2Cl_2$ zugetropft. Man läßt auf RT kommen, wäscht mit Wasser, 1N Salzsäure und gesättigter $NaHCO_3$-Lösung, trocknet über $MgSO_4$ und engt ein. Der Feststoff wird an 300 g Kieselgel durch Flash-Chromatographie mit $CH_2Cl_2$:MeOH = 97:3 als Eluent gereinigt.

Ausbeute: 9 g = 50%
Fp: 174°C
$[\alpha]_D$: -20,3° (c=1, $CHCl_3$)

2) Darstellung der optisch aktiven S-Oxide enthaltenden Aminosäurederivate der Formel (I) durch Oxidation optisch aktiver schwefelhaltiger Aminosäurederivate der Formel (IV)

Beispiel 3

a) L-Methionin-l-menthylester
0,3 mol L-Methionin, 0,39 mol (-)-Menthol und 0,33 mol p-Toluolsulfonsäure-Monohydrat werden in 1,2 l Toluol am Wasserabscheider erhitzt. Die Badtemperatur sollte 140°C nicht überschreiten. Nach 30 h hat sich eine klare Lösung gebildet, und es wurden 10 ml Wasser abgeschieden (Theorie: 11,4 ml).

Nach dem Abkühlen bringt man mit 15%iger Natronlauge auf pH 11 bis 12, trennt die Toluolphase ab und extrahiert die wäßrige Phase noch zweimal mit Toluol. Die vereinigten Toluolphasen werden einmal mit Wasser gewaschen, über Magnesiumsulfat getrocknet und eingeengt. Aus dem Rohprodukt wird überschüssiges (-)-Menthol unter vermindertem Druck abdestilliert.
Ausbeute (Destillationsrückstand): 71 g ≙ 82% $[\alpha]_D$ = -38,1° (c = 1, $CHCl_3$)

b) N-Methacryloyl-L-methionin-l-menthylester

60 mmol L-Methionin-l-menthylester werden in 500 ml trockenem Dichlormethan bei 0°C mit 66 mmol Triethylamin versetzt. Man kühlt auf -10°C ab und tropft 65 mmol frisch destilliertes Methacryloylchlorid in 50 ml Dichlormethan zu. Man läßt auf Raumtemperatur kommen und rührt noch 1 h nach. Man wäscht mit Wasser, 1N-Salzsäure und gesättigter Natriumhydrogencarbonat-Lösung, trocknet über Magnesiumsulfat und engt ein. Das Rohprodukt wird aus n-Heptan umkristallisiert.
Ausbeute: 17,3 g ≙ 81%
Fp: 80-82°C
$[\alpha]_D$ = -27,3° (c = 1, $CHCl_3$)

c) N-Methacryloyl-L-methionin-l-menthylester-(R,S)-sulfoxid

20 mmol N-Methacryloyl-L-methionin-l-menthylester in 40 ml Methanol werden zu 42 ml einer 0,5 M Lösung von Natriummetaperiodat in Wasser bei 0°C getropft. Nach 6 h wird ausgefallenes Natriumiodat abgesaugt. Das Filtrat wird mehrfach mit Chloroform extrahiert. Man trocknet über Magnesiumsulfat und engt ein. Das Rohprodukt kristallisiert durch und wird aus n- Heptan umkristallisiert.
Ausbeute: 6 g ≙ 81%
Fp: 79°C
$[\alpha]_D$ = 33,4° (c=1, $CHCl_3$)
$^1H$-NMR ($CDCl_3$): zeigt 2 Diastereomere im Verhältnis 1:1

Beispiel 4

N-Methacryloyl-L-methionin-l-menthylester-sulfon

15 mmol N-Methacryloyl-L-methionin-l-menthylester (vgl. Beispiel 3b)) werden in 60 ml Eisessig mit 2,4 Äquivalenten 35%igem Wasserstoffperoxid versetzt. Man rührt 8 h bei 50°C nach. Die Reaktionslösung wird bei RT eingeengt. Das Rohprodukt wird aus n-Heptan umkristallisiert.
Ausbeute: 5,35 g = 92%
Fp: 141°C
$[\alpha]_D$ = -18,6° (c=1, $CHCl_3$)

Beispiel 5

a) N-BOC-L-methionin-l-menthylamid

0,45 mol N-BOC-L-methionin in 1 l THF werden bei 0°C mit 0,45 mol Triethylamin versetzt. Man kühlt auf -12°C ab und tropft 0,45 mol Chlorkohlensäureethylester zu. Man läßt 20 min bei -12°C nachrühren und tropft 0,45 mol L-Menthylamin zu. Man läßt 45 min bei 0°C nachrühren und dann auf Raumtemperatur kommen.

Das THF wird abdestilliert; der Rückstand wird in 1 l Dichlormethan aufgenommen und mit Wasser und 1N-Salzsäure gewaschen, über Magnesiumsulfat getrocknet und erneut eingeengt.
Rohausbeute: 170 g = 98%
(enthält laut GC ~ 14% N-Menthyl-ethylurethan).

b) L-Methionin-l-menthylamid

0,4 mol N-BOC-L-methionin-l-menthylamid werden in 300 ml Dichlormethan bei 0°C mit 300 ml Trifluoressigsäure versetzt. Man läßt auf Raumtemperatur kommen und rührt 1 h nach. Der Ansatz wird eingeengt und erneut in 500 ml Dichlormethan aufgenommen. Unter Kühlung wird mit 15%iger Natronlauge auf pH 12 eingestellt. Die organische Phase wird abgetrennt. Die wäßrige Phase wird noch zweimal mit Dichlormethan extrahiert. Die vereinigten organischen Phasen werden mit Wasser gewaschen, über Magnesiumsulfat getrocknet und eingeengt. Rohausbeute: 102 g = 89% (enthält noch Urethan, s.o.)

c) N-Methacryloyl-L-methionin-l-menthylamid

134 mmol L-Methionin-l-menthylamid werden in 1,2 l Dichlormethan bei 0°C mit 134 mmol Triethylamin versetzt. Nach Abkühlen auf -10°C tropft man 134 mmol frisch destilliertes Methacryloylchlorid zu. Man läßt auf Raumtemperatur kommen und wäscht mit Wasser, 1N-Salzsäure und gesättigter Natriumhydrogencarbonat-Lösung. Nach Trocknen über Magnesiumsulfat wird das Lösungsmittel abdestilliert. Das kristalline Rohprodukt wird aus n-Heptan umkristallisiert.

Ausbeute: 37,3 g = 79%
Fp: 161-163°C
$[\alpha]_D$ = -63,4° (c = 1, $CHCl_3$)

d) N-Methacryloyl-L-methionin-l-menthylamid-(R,S)-sulfoxid
19 mmol N-Methacryloyl-L-methionin-l-menthylamid werden in 50 ml Ethanol mit 1,2 Äquivalenten 35%igem Wasserstoffperoxid bei RT versetzt. Man läßt 18 h nachrühren. Die Reaktionslösung wird bei RT eingeengt. Das Rohprodukt wird aus tert.-Butyl-methylether kristallisiert.
Ausbeute: 5,9 g ≙ 84%
Fp: 155-158°C
$[\alpha]_D$ = -67,4° (c = 1, $CHCl_3$)
$^1$H-NMR ($CDCl_3$): zeigt 2 Diastereomere im Verhältnis 1:1.

Beispiel 6

N-Methacryloyl-L-methionin-l-menthylamid-sulfon

15 mmol N-Methacryloyl-L-methionin-l-menthylamid (vgl. Beispiel 5c)) werden in 75 ml Eisessig mit 2,4 Äquivalenten 35%igem Wasserstoffperoxid versetzt und 18 h bei 50°C nachgerührt. Nach Einengen bei Raumtemperatur wird aus tert.-Butylmethylether kristallisiert.
Ausbeute: 2,25 g ≙ 39%
Fp: 156°C
$[\alpha]_D$ = -58,2° (c = 1, $CHCl_3$)

Beispiel 7

a) N-Methacryloyl-S-methyl-L-cystein
0,2 Mol S-Methyl-L-cystein werden bei 0°C in 100 ml 2N Natronlauge eingetragen. 0,2 Mol frisch destilliertes Methacryloylchlorid - mit Hydrochinon stabilisiert - werden parallel mit weiteren 100 ml 2N Natronlauge so zugetropft, daß T = 0°C und pH 11 beibehalten wird. 1h nachgerührt, mit 85%iger ortho-Phosphorsäure unter starkem Rühren auf pH 2 gebracht, mit festem Natriumchlorid gesättigt und mit Essigester extrahiert. Die Extrakte werden mit Magnesiumsulfat getrocknet und eingeengt (stabilisiert mit Hydrochinon).
Ausbeute: 40 g = 99%
$[\alpha]_D$ = +29,3° (c = 1, $CHCl_3$)

b) N-Methacryloyl-S-methyl-L-cystein-l-menthylamid
Zu 0,2 Mol N-Methacryloyl-S-methyl-L-cystein in 400 ml THF werden bei 5°C 0,2 Mol L-Menthylamin in 300 ml THF zugetropft. Dann setzt man 0,2 Mol EEDQ in 500 ml THF zu. Man läßt 4 Tage bei RT nachrühren. Man engt ein und nimmt in 700 ml Essigester auf, wäscht 5 x mit 1N Salzsäure, 1 x mit Wasser, 1 x mit gesättigter Kochsalzlösung, trocknet über Magnesiumsulfat und engt ein. Das Rohprodukt wird aus tert.-Butylmethylether kristallisiert.
Ausbeute: 40 g = 58,5%
Fp: 153°C
$[\alpha]_D$ = -81,4° (c = 1, MeOH)

c) N-Methacryloyl-S-methyl-L-cystein-l-menthylamid-sulfon
120 mmol N-Methacryloyl-S-methyl-L-cystein-l-menthylamid werden in 400 ml Aceton suspendiert, mit 140 mmol Tripelsalz der Caro'schen Säure ($2KHSO_5xKHSO4xK_2SO_4$) in 400 ml Wasser versetzt und bei 40°C gerührt. Nach Ende der Reaktion wird mit 10%iger Natriumbisulfit-Lösung überschüssiges Peroxid zerstört. Aceton wird abgezogen. Die wäßrige Phase wird mit Essigester extrahiert. Die organische Phase wird über Magnesiumsulfat getrocknet und eingeengt.
Ausbeute: 41,5 g = 93%
Fp: 199°C
$[\alpha]_D$ = -68,9° (c = 1, $CHCl_3$).

Beispiel 8

N-Methacryloyl-S-methyl-L-cystein-l-menthylamid-(R,S)-sulfoxid

140 mmol N-Methacryloyl-S-methyl-L-cystein-l-menthylamid (vgl. Beispiel 7b)) werden in 500 ml Eisessig vorgelegt. Bei 15 bis 20°C werden 1,2 Äquivalente 35%iges Wasserstoffperoxid zugetropft. Nach Reaktionsende wird bei Raumtemperatur eingeengt. Das Rohprodukt wird aus tert.-Butylmethylether kristallisiert.
Ausbeute: 33 g = 66%
Fp: 194°C
$[\alpha]_D$: -101,4° (c = 1, $CHCl_3$)
$^1$H-NMR ($CDCl_3$): zeigt 2 Diastereomere im Verhältnis 1:1.

Beispiel 24 und 25

a) (R)-Thioprolin-l-menthylester
0,2 Mol (R)-Thioprolin, 0,256 Mol l-Menthol und 0,221 Mol p-Toluolsulfonsäure-monohydrat werden in 1 l Toluol am Wasserabscheider refluxiert, bis die theoretische Menge Reaktionswasser abgeschieden ist. Man stellt mit 15%iger Natronlauge unter Kühlung auf pH 11-12 und extrahiert 3 x mit Toluol. Die organische Phase wird einmal mit Wasser gegengewaschen, über Magnesiumsulfat getrocknet und eingeengt. Überschüssiges l-Menthol wird aus dem Rückstand im Vakuum abdestilliert.
Ausbeute: 31 g = 57%
$[\alpha]_D$ = -129,0° (c = 1, $CHCl_3$).

b) (R)-N-Acryloyl-thioprolin-l-menthylester
0,1 Mol (R)-Thioprolin-l-menthylester in 800 ml Dichlormethan werden bei 0°C mit 0,1 Mol Triethylamin versetzt. Dann werden bei -10°C 0,1 Mol Acryloylchlorid in 70 ml Dichlormethan zugetropft. Man läßt 1 h bei Raumtemperatur nachrühren. Man wäscht mit Wasser, 1N Salzsäure und gesättigter Natriumhydrogencarbonatlösung, trocknet über Magnesiumsulfat und engt ein.
Ausbeute: 31,5 g = 97%
$[\alpha]_D$ = -124,9° (c = 1, $CHCl_3$)

c) (R)-N-Acryloyl-thioprolin-l-menthylester-(R)-sulfoxid (Bsp. 24c) und -(S)-sulfoxid (Bsp. 25c)
72 mmol (R)-N-Acryloyl-thioprolin-l-menthylester werden in 200 ml Ethanol:Eisessig=1:1 mit 1,2 Äquivalenten 35%igem Wasserstoffperoxid versetzt. Man läßt 8 h bei Raumtemperatur nachrühren. Die Reaktionslösung wird eingeengt. Das Rohprodukt, das im DC 2 unterscheidbare Produktflecken zeigt ($^t$BuOMe:MeOH=95:5 als Laufmittel), wird mit eben genanntem Eluenten an Kieselgel chromatographiert.
Ausbeute: 11,3 g = 46% 1. Fraktion; $[\alpha]_D$ = -184,7° (c = 1, $CHCl_3$) = Bps. 24c (R)-Sulfoxid 3,0 g = 12% 2. Fraktion; $[\alpha]_D$ = -95,5° (c = 1, $CHCl_3$) = Bsp. 25c (S)-Sulfoxid

<u>Tabelle 1</u>: Monomersynthese

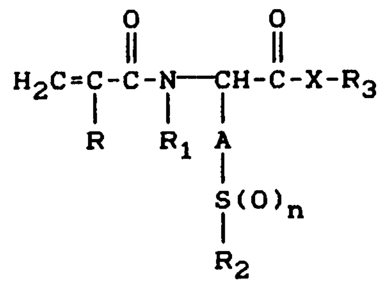

| Beispiel | Rest-Gruppen | Produkt-Name | Fp ($^{\circ}C$) | $[\alpha]_D$ (c=1, $CHCl_3$) | Ausbeute (%) |
|---|---|---|---|---|---|
| 1e | $R=CH_3$, $R_1=H$, $R_2=CH_3$ $R_3$=l-menthyl, X=NH, $A=CH_2CH_2$ n=1 | N-Methacryloyl-L-methionin-l-menthylamid(S)-sulfoxid | 174 | -20,3 | 50 |
| 2 | wie 1e | N-Methacryloyl-L-methionin-l-menthylamid(R)-sulfoxid | 151 | -75,0 | 58 |

Tabelle 1: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 3c | $R=CH_3$, $R_1=H$, $R_2=CH_3$, $R_3$= l-menthyl, X=O $A=CH_2CH_2$, n=1 | N-Methacryloyl-L-methionin-l-menthylester(R,S)-sulfoxid | 79 | -33,4 | 81 |
| 4 | wie 3c aber: n=2 | N-Methacryloyl-L-methionin-l-menthylestersulfon | 141 | -18,6 | 92 |
| 5d | $R=CH_3$, $R_1=H$, $R_2=CH_3$ $R_3$=l-menthyl, X=NH $A=CH_2CH_2$, n=1 | N-Methacryloyl-L-methionin l-menthylamid(R,S)-sulfoxid | 155-158 | -67,4 | 84 |
| 6 | wie 5d aber: n=2 | N-Methacryloyl-L-methionin-l-menthylamidsulfon | 156 | -58,2 | 39 |

Tabelle 1: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 7c | $R=CH_3$, $R_1=H$, $R_2=CH_3$ $R_3$=l-menthyl, X=NH $A=CH_2$, n=2 | N-Methacryloyl-S-methyl-L-cystein-l-menthylamidsulfon | 199 | -68,9 | 93 |
| 8 | wie 7c aber: n=1 | N-Methacryloyl-S-methyl-L-cystein-l-menthylamid(R,S)-sulfoxid | 194 | -101,4 | 66 |
| 9 | R=H, $R_1=H$, $R_2=CH_3$ $R_3$=l-menthyl, X=O $A=CH_2CH_2$, n=1 | N-Acryloyl-L-methionin-l-menthylester(R,S)-sulfoxid | 90 | -23,1 | 62 |
| 10 | wie 9 aber: X=NH | N-Acryloyl-L-methionin-l-menthylamid(R,S)-sulfoxid | 173 | -83,4 | 82 |

Tabelle 1: Fortsetzung

| Beispiel | Rest-Gruppen | Produkt-Name | Fp (°C) | $[\alpha]_D$ ($c=1$, $CHCl_3$) | Ausbeute (%) |
|---|---|---|---|---|---|
| 11 | wie 9 aber: $R_3$=d-menthyl | N-Acryloyl-L-methionin-d-menthylester-(R,S)-sulfoxid | 82 | +62,0 | 84 |
| 12 | $R=CH_3$, $R_1=H$, $R_2=CH_3$ $R_3$=d-menthyl, X=O, $A=CH_2CH_2$, n=1 | N-Methacryloyl-L-methionin-d-menthylester-(R,S)-sulfoxid | 61 | +48,6 | 66 |
| 13 | wie 12 aber: X=NH | N-Methacryloyl-L-methionin-d-menthylamid(R,S)-sulfoxid | 134 | +29,8 | 78 |
| 14 | R=H, $R_1=H$, $R_2=CH_3$ $R_3$=d-menthyl, X=NH $A=CH_2CH_2$, n=1 | N-Acryloyl-L-methionin-d-menthylamid-(R,S)-sulfoxid | 167 | +13,2 | 93 |

<u>Tabelle 1</u>: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 15 | wie 14 aber: n=2 | N-Acryloyl-L-methionin-d-menthylamid-sulfon | 164 | +17,1 | 79 |
| 16 | R=H, $R_1$=H, $R_2$=$CH_3$ $R_3$=l-menthyl, X=O A=$CH_2CH_2$,n=2 | N-Acryloyl-L-methionin-l-menthylester-sulfon | 140 | -11,0 | 95 |
| 17 | R=H, $R_1$=H, $R_2$=$CH_3$ $R_3$=d-menthyl, X=O A=$CH_2CH_2$, n=2 | N-Acryloyl-L-methionin-d-menthyl-estersulfon | 156 | +70,8 | 93 |
| 18 | R=H, $R_1$=H, $R_2$=$CH_3$ $R_3$=l-menthyl, X=NH A=$CH_2CH_2$, n=2 | N-Acryloyl-L-methionin-l-menthyl-amidsulfon | 188 | -54,1 | 71 |

Tabelle 1: Fortsetzung

| Beispiel | Rest-Gruppen | Produkt-Name | Fp ($^{o}C$) | $[\alpha]_D$ (c=1, $CHCl_3$) | Ausbeute (%) |
|---|---|---|---|---|---|
| 19 | $R=CH_3$, $R_1=H$, $R_2=CH_3$ $R_3$=d-menthyl, X=NH $A=CH_2CH_2$, n=2 | N-Methacryloyl-L-methionin-d-menthylamidsulfon | 147 | +33,1 | 77 |
| 20 | $R=CH_3$, $R_1=H$, $R_2=CH_3$ $R_3$=d-menthyl, X=O $A=CH_2CH_2$, n=2 | N-Methacryloyl-L-methionin-d-menthylestersulfon | 110 | +32,7 (THF) | 80 |
| 21 | R=H, $R_2R_1=CH_2$ $R_3$=l-menthyl, X=NH $A=CH_2$, n=1 | (R)-N-Acryloyl-thioprolin-l-menthylamid(R)-sulfoxid | 100 | -244,8 | 71 |
| 22 | R=H, $R_2R_1=CH_2$ $R_3$=d-menthyl, X=O $A=CH_2$, n=1 | (R)-N-Acryloyl-thioprolin-d-menthylester(R)-sulfoxid | Öl | -117,4 | 35 |

<u>Tabelle 1</u>: Fortsetzung

| Beispiel | Rest-Gruppen | Produkt-Name | Fp (°C) | $[\alpha]_D$ ($c=1$, $CHCl_3$) | Ausbeute (%) |
|---|---|---|---|---|---|
| 23 | R=H, $R_1$=H, $R_2$=$CH_3$ $R_3$=Et, X=O A=$CH_2CH_2$, n=2 | N-Acryloyl-L-methioninethylester-sulfon | 115 | +58,0 | 89 |
| 24c | R=H, $R_2R_1$=$CH_2$ $R_3$=l-menthyl, X=O A=$CH_2$, n=1 | (R)-N-Acryloyl-thioprolin-l-menthylester(R)-sulfoxid | Öl | -184,7 | 46 |
| 25c | wie 24 | (R)-N-Acryloyl-thioprolin-l-menthyl-ester(S)-sulfoxid | Öl | -95,5 | 12 |
| 26 | R=H, $R_2R_1$=$CH_2$ $R_3$=d-menthyl, X=NH A=$CH_2$, n=1 | (R)-N-Acryloyl-thioprolin-l-menthyl-amid(R)-sulfoxid | 127 | -127,2 | 61 |

Tabelle 1: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 27 | R=F, $R_1$=H, $R_1$=$CH_3$ $R_3$=(1S)-bornyl, X=O A=$CH_2CH_2$, n=1 | N-Fluoracryloyl-L-methionin(1S)-bornylester(R,S)-sulfoxid | Öl | +2,0 | 52 |
| 28 | wie 27 aber n=2 | N-Fluoracryloyl-L-methionin(1S)-bornylestersulfon | 101 | +11,5 | 80 |
| 29 | R=$CH_3$, $R_1$=H, $R_2$=$CH_3$ $R_3$=tert.-butyl, X=O A=$CH_2CH_2$, n=2 | N-Methacryloyl-L-methionin-tert.-butylestersulfon | 99 | +25,3 | 79 |
| 30 | R=H, $R_1$=H, $R_2$=$CH_3$ $R_3$=(1S)-bornyl, X=O A=$CH_2CH_2$, n=2 | N-Acryloyl-L-methionin(1S)-bornylestersulfon | Öl | +3,8 | 78 |

<u>Tabelle 1</u>: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 31 | wie 30 aber $R=CH_3$ | N-Methacryloyl-L-methionin(1S)-bornylestersulfon | 110 | -6,6 | 69 |
| 32 | $R=H$, $R_2R_1=CH_2$ $R_3$=3,5-Dimethylphenyl $X=NH$, $A=CH_2$, $n=1$ | (R)-N-Acryloyl-thioprolin-3,5-dimethylanilid-(R)-sulfoxid | 204 | -240,9 | 52 |
| 33 | $R=CH_3$, $R_1=H$, $R_2=CH_3$ $R_3$=(S)-Phenylethyl $X=NH$, $A=CH_2CH_2$, $n=2$ | N-Methacryloyl-L-methionin-(S)-phenylethylamid-sulfon | 125 | -117,7 | 74 |
| 34 | $R=CH_3$, $R_1=H$, $R_2=CH_3$ $R_3$=Phenyl, $X=NH$ $A=CH_2CH_2$, $n=2$ | N-Methacryloyl-L-methionin-phenylamid-sulfon | 137 | -20,3 | 83 |

Tabelle 1: Fortsetzung

| Beispiel | Rest-Gruppen | Produkt-Name | Fp (°C) | $[\alpha]_D$ (c=1, $CHCl_3$) | Ausbeute (%) |
|---|---|---|---|---|---|
| 35 | $R=CH_3$, $R_1=H$, $R_2=CH_3$ $R_3$=3-Pentyl, X=NH $A=CH_2CH_2$, n=2 | N-Methacryloyl-L-methionin-3-pentylamidsulfon | 134 | +8,6 (MeOH) | 94 |
| 36 | $R=CH_3$, $R_1=H$, $R_2=CH_3$ $R_3$=Ethyl, X=NEt $A=CH_2$, n=2 | N-Methacryloyl-S-methyl-L-cystein-diethylamidsulfon | Öl | -21,1 | 65 |
| 37 | $R=CH_3$, $R_1=H$, $R_2=CH_3$ $R_3$=1-menthyl, X=O $A=CH_2$, n=2 | N-Methacryloyl-S-methyl-L-cystein-1-menthylestersulfon | 128 | -39,8 | 56 |
| 38 | wie 37 aber: n=1 | N-Methacryloyl-S-methyl-L-cystein-1-menthylester(R,S)-sulfoxid | 120 | -46,8 | 59 |

Tabelle 1: Fortsetzung

| Beispiel | Rest-Gruppen | Produkt-Name | Fp ($^0$C) | $[\alpha]_D$ (c=1, $CHCl_3$) | Ausbeute (%) |
|---|---|---|---|---|---|
| 39 | $R=CH_3$, $R_1=H$, $R_2=CH_2Ph$ $R_3$=1-menthyl, X=NH $A=CH_2$, n=2 | N-Methacryloyl-S-benzyl-L-cystein-1-menthylamidsulfon | 185 | - 34,0 | 69 |
| 40 | $R=CH_3$, $R_1=H$, $R_2=CH_2Ph$ $R_3$=1-menthyl, X=NH $A=CH_2$, n=1 | N-Methacryloyl-S-benzyl-L-cystein-1-menthylamid-(R,S)-sulfoxid | 203-205 | - 49,9 | 85 |
| 41 | $R=CH_3$, $R_1=H$, $R_2=CH_3$ $R_3$=3-pentyl, X=NH $A=CH_2$, n=1 | N-Methacryloyl-S-methyl-L-cystein-3-pentylamid-(R,S)-sulfoxid | 152 | - 51,9 | 96 |
| 42 | $R=CH_3$, $R_1=H$, $R_2=CH_3$ $R_3$=3-pentyl, X=NH $A=CH_2$, n=2 | N-Methacryloyl-5-methyl-L-cystein-3-pentylamidsulfon | 144 | - 34,0 | 57 |

Tabelle 1: Fortsetzung

| Beispiel | Rest-Gruppen | Produkt-Name | Fp (°C) | $[\alpha]_D$ ($c=1$, $CHCl_3$) | Ausbeute (%) |
|---|---|---|---|---|---|
| 43 | $R=CH_3$, $R_1=H$, $R_2=CH_3$<br>$R_3$=(S)-Phenylethyl<br>X=NH, $A=CH_2$, n=1 | N-Methacryloyl-S-methyl-L-cystein-(S)-phenylethylamid-(R,S)-sulfoxid | 161 | - 80,8 | 91 |
| 44 | $R=CH_3$, $R_1=H$, $R_2=CH_3$<br>$R_3$=3-Methylenylpinanyl<br>X=NH, $A=CH_2$, n=1 | N-Methacryloyl-S-methyl-L-cystein-3-(3S)-pinanylmethylamid-(R,S)-sulfoxid | 123-125 | - 20,8 | 64 |
| 45 | $R=CH_3$, $R_1=H$, $R_2=CH_3$<br>$R_3$=(R)-Phenylethyl<br>X=NH, $A=CH_2$, n=1 | N-Methacryloyl-S-methyl-L-cystein-(R)-phenylethylamid-(R,S)-sulfoxid | 164-165 | - 41,9 | 74 |
| 46 | $R=CH_3$, $R_1=H$, $R_2=CH_3$<br>$R_3$=d-Neomenthyl<br>X=NH, $A=CH_2$, n=1 | N-Methacryloyl-S-methyl-L-cystein-d-neomenthylamid-(R,S)-sulfoxid | 138-140 | - 27,1 | 55 |

3) Polymerisation der optisch aktiven S-Oxide enthaltenden Aminosäurederivate der Formel I auf Kieselgel

a) Herstellung von Vinylsilica

Unter strengem Feuchtigkeitsausschluß werden 50 g bei 120°C getrocknetes Kieselgel (LiCHrosorb Si 100, 5 μ, Merck; Polygosil 100-5, Macherey & Nagel) mit 600 ml trockenem Toluol versetzt. 100 ml Toluol werden bei Normaldruck unter $N_2$ abdestilliert.

Nach Abkühlen auf 30°C werden 35 g Trichlorvinylsilan zugegeben. Der Ansatz wird unter Rühren (KPG-Rührer) zum Rückfluß erhitzt. Dann werden 62 g Triethylamin in 150 ml Toluol innerhalb einer Stunde zugetropft. Man läßt über Nacht bei 105-108°C weiterrühren.

Nach Abkühlen und Absaugen über eine Glasfritte wird das Kieselgel nacheinander in Toluol, Dichlormethan, Methanol, Methanol/Wasser = 60/40, zweimal Methanol und zweimal Dichlormethan ausgerührt, zwischendurch jeweils gründlich trockengesaugt und am Ende bei 70°C getrocknet.

Analysen: C: 2,8-3,2%; H:0,7-0,9%

b) Herstellung der chiralen Kieselgele

3,0 g Vinylsilica, 2,0 g Monomer (siehe Tabelle 2) und 40 mg Azobisisobutyronitril werden in 8 ml Toluol, Toluol/Heptan-Gemischen oder Chloroform gelöst bzw. suspendiert. Unter Magnetrührung wird die Apparatur dreimal evakuiert und mit Stickstoff belüftet, Die Mischung wird 1 Stunde bei Raumtemperatur gerührt, dann rasch auf 80°C (bzw. 55°C für Chloroform) erwärmt und 45 min bei dieser Temperatur belassen, Nach Zusatz von 100 mg 2,6-Di-tert.-butyl-4-methylphenol wird rasch auf Raumtemperatur gekühlt. Das Kieselgel wird über eine G4-Fritte abgesaugt, zweimal in Chloroform, einmal in Toluol und einmal in Isopropanol je 15 min ausgerührt, gut trockengesaugt und bei Raumtemperatur im Vakuum (<0,005 atm) getrocknet.

Ausbeute; 3,35 g ± 10%

Belegungsgrad siehe Tabelle 2.

Tabelle 2: Kieselgelphasen

| Beispiel | Monomer | Lösungsmittel | N-Gehalt [%] | gebundenes Polymer [Gew.-%] |
|---|---|---|---|---|
| 1A | 1e | $CHCl_3$ | 1,0 | 13,2 |
| 2A | 2 | $CHCl_3$ | 1,2 | 15,9 |
| 3A | 3c | Toluol | 0,7 | 18,6 |
| 4A | 4 | Toluol | 0,55 | 15,2 |
| 5A | 5d | $CHCl_3$ | 0,75 | 9,9 |
| 6A | 6 | Toluol | 1,2 | 16,6 |
| 7A | 7c | $CHCl_3$ | 0,8 | 10,7 |
| 8A | 8 | $CHCl_3$ | 0,7 | 8,9 |
| 9A | 9 | $CHCl_3$ | 0,5 | 12,8 |
| 10A | 10 | Toluol | 1,3 | 16,6 |
| 11A | 11 | $CHCl_3$ | 0,55 | 14,0 |
| 12A | 12 | Toluol | 0,65 | 17,3 |
| 13A | 13 | Toluol | 1,5 | 19,9 |
| 14A | 14 | Toluol | 1,2 | 15,3 |
| 15A | 15 | $CHCl_3$ | 1,05 | 14,0 |
| 16A | 16 | $CHCl_3$ | 0,35 | 9,3 |
| 17A | 17 | $CHCl_3$ | 0,6 | 16,0 |
| 18A | 18 | Toluol | 1,55 | 20,6 |
| 19A | 19 | Toluol | 0,8 | 11,0 |
| 20A | 20 | Toluol | 0,75 | 20,8 |
| 21A | 21 | Toluol | 0,7 | 8,5 |
| 22A | 22 | Toluol | 0,5 | 12,2 |
| 23A | 23 | Toluol | 1,25 | 23,5 |

Tabelle 2: Fortsetzung

| Beispiel | Monomer | Lösungsmittel | N-Gehalt [%] | gebundenes Polymer [Gew.-%] |
|---|---|---|---|---|
| 24A | 24c | Toluol | 0,65 | 15,9 |
| 25A | 25c | Toluol | 0,6 | 14,6 |
| 27A | 27 | Toluol | 0,6 | 16,0 |
| 28A | 28 | Toluol | 0,5 | 13,9 |
| 29A | 29 | Toluol | 1,3 | 28,3 |
| 30A | 30 | Toluol | 0,65 | 17,3 |
| 31A | 31 | $CHCl_3$ | 0,4 | 11,0 |
| 32A | 32 | $CHCl_3$ | 0,9 | 9,8 |
| 33A | 33 | $CHCl_3$ | 1,25 | 15,7 |
| 34A | 34 | $CHCl_3$ | 1,7 | 19,7 |
| 35A | 35 | $CHCl_3$ | 2,0 | 22,8 |
| 36A | 36 | Toluol/Heptan (mit 0,5 g Monomer 36) | 1,2 | 12,4 |
| 37A | 37 | Toluol | 0,3 | 8,0 |
| 38A | 38 | Toluol | 0,45 | 11,5 |
| 39A | 39 | $CHCl_3$ | 0,85 | 13,6 |
| 40A | 40 | $CHCl_3$ | 0,75 | 11,6 |
| 41A | 41 | $CHCl_3$ | 1,9 | 19,6 |
| 42A | 42 | $CHCl_3$ | 1,6 | 17,4 |

4) Polymerisation der optisch aktiven S-Oxide enthaltenden Aminosäurederivate der Formel I zu Perlpolymerisaten

Beispiel 7B:

In eine 250 ml-Rührapparatur werden 13,0 g N-Methacryloyl-S-methyl-L-cystein-l-menthylamid-sulfon (vgl. Monomerbeispiel 7c)), 2,0 g N,N',N''-Tris(acryloyl)perhydrotriazin (Triacrylformal), 0,5 g Azobisisobutyronitril, 45 g Trichlormethan und eine Lösung von 4 g Polyvinylalkohol in 130 ml 1M Phosphatpuffer pH 6,0 eingefüllt. Die Apparatur wird mehrmals evakuiert und mit Stickstoff wieder befüllt. Die Mischung wird dann unter Stickstoff mit 425 U/min gerührt und 15 min bei 45°C, 30 min bei 50°C und 14 Stunden bei 55°C polymerisiert. Die Feinstanteile <10µm werden durch mehrmaliges Sedimentieren in Wasser abgetrennt, die Perlen abgesaugt, mit Aceton, Trichlormethan und wieder Aceton intensiv gewaschen und bei 50°C getrocknet.
Ausbeute: 13 g Perlpolymerisat
Korngröße: 10-40µm

Schüttvolumen: 2,0 ml/g
Quellvolumen: 4,5 ml/g (in Toluol/THF=3/2 (V/V))

Tabelle 3

| Perlpolymerisate | | | | | |
|---|---|---|---|---|---|
| Beispiel | Monomer | Vernetzer | Korngröße | Vs [ml/g] | Vg [ml/g] |
| 7B | 7c | 13,3% Triacrylformal | 10-40 | 2,0 | 4,5 |
| 8B | 8 | 13,3% Triacrylformal | 10-80 | 1,7 | 4,7 |

5) Verwendung der optisch aktiven Polymerisate der S-Oxide enthaltenden Aminosäurederivate I als Adsorbentien für die Racemattrennung

Für die chromatographischen Trennungen wurden folgende Testracemate verwendet:

Racemat Nr. 1: Binaphthol
Racemat Nr. 2: Chlorthalidon
Racemat Nr. 3: Mandelsäureamid
Racemat Nr. 4: Ibuprofenamid
Racemat Nr. 5: Trifluoranthrylethanol
Racemat Nr. 6: Benzoin
Racemat Nr. 7: Thiaprofen

Die an Kieselgel gebundenen Polymerisate wurden in Stahlsäulen (Innendurchmesser: 4 mm; Länge; 25 cm) unter HPLC-Bedingungen eingesetzt. Eluiert wurde mit n-Heptan-Tetrahydrofurangemischen (z.B.: 1:1 und 1:4 (Vol/Vol)) oder n-Heptan-Isopropanolgemischen (z.B.: 10:1 (Vol/Vol)). Die Fließmittelgeschwindigkeit betrug 1 ml/min.

Die Perlpolymerisate wurden in einer Glassäule (Innendurchmesser: 1,2 cm; Betthöhe: 25-30 cm) unter Niederdruckbedingungen eingesetzt. Eluiert wurde mit einem Toluol-Tetrahydrofurangemisch = 3:1 (Vol/Vol). Die Fließmittelgeschwindigkeit betrug 0,5 ml/min.

Die bei der chromatographischen Trennung der verschiedenen Testracemate erhaltenen Ergebnisse (Enantioselektivität $\alpha$ und Kapazitätsverhältnis $k_1$') und die verwendeten Elutionsmittel sind in den Tabellen 4 und 5 zusammengestellt. In Tabelle 4 werden zwei Beispiele für die erfindungsgemäßen Adsorbentien (n=1 und n=2 in der Monomerformel I) im Vergleich mit der analogen Phase mit n=0 (Monomerformel I) gegeben. Es zeigt sich, daß die S-Oxid-Phasen den Sulfid-Phasen nicht nur überlegen hinsichtlich der Enantioselektivität $\alpha$ sind, sondern auch sehr überraschend nach einem anderen Trennmechanismus arbeiten, wie an der Umkehrung der Elutionsreihenfolge der Enantionmere der Testracemate Nr. 2 und Nr. 3 zu sehen ist ((-) vor (+) heißt, daß das (-)-drehende Enantiomer vor dem (+)-drehenden Enantiomer

(Polarimeter) eluiert wird).

Tabelle 4: Trennergebnisse für Monomerbeispiele 7c (n=2) und 8 (n=1) (HPLC)

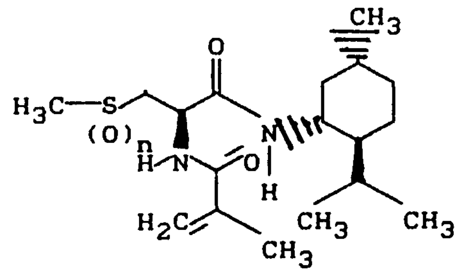

α = Enantioselektivität

$k_1$' = Kapazitätsverhältnis

(-), (+) = Elutionsreihenfolge

| Racemat | n=0 (Sulfid) | n= 1 (Sulfoxid) | n=2 (Sulfon) | Elutionsmittel |
|---|---|---|---|---|
| Nr. 1 | α= 1,23<br>$k_1$'= 0,47 | α=2,28<br>$k_1$'=1,08 | α=2,46<br>$k_1$'=0,40 | n-Heptan: THF = 1:1 |
| Nr. 2 | α= 1,84<br>$k_1$'= 0,47<br>(+) vor (-) | α=4,59<br>$k_1$'=1,08<br>(-) vor (+) | α=3,80<br>$k_1$'=0,30<br>(-) vor (+) | n-Heptan: THF = 1:4 |
| Nr. 3 | α= 1,23<br>$k_1$'= 0,95<br>(+) vor (-) | α=2,57<br>$k_1$'=0,64<br>(-) vor (+) | α=1,78<br>$k_1$'=0,62<br>(-) vor (+) | n-Heptan: THF = 1:4 |
| Nr. 4 | α= 1,38<br>$k_1$'= 1,62 | α=1,88<br>$k_1$'=1;14 | α=1,54<br>$k_1$'=0,93 | n-Heptan: THF = 1:1 |
| Nr. 5 | α= 1,00<br>keine Trennung | α=1,20<br>$k_1$'=1,73 | α=1,09<br>$k_1$'=1,01 | n-Heptan: Isopropanol= 10:1 |
| Nr. 6 | α= 1,00<br>keine Trennung | α=1,00<br>keine Trennung | α=1,56<br>$k_1$'=1,20 | n-Heptan: Isopropanol = 10:1 |

Tabelle 4 - Fortsetzung

| Racemat | n=0 (Sulfid) | n= 1 (Sulfoxid) | n=2 (Sulfon) | Elutionsmittel |
|---|---|---|---|---|
| Nr. 7 | $\alpha$= 1,00<br>keine Trennung | $\alpha$=1,37<br>$k_1'$=0,80<br>(+) vor (-) | d=1,00<br>keine Trennung | n-Heptan: THF<br>= 1:1 |

<u>Tabelle 5</u>: Trennergebnisse für Monomerbeispiel 40 (n=1)

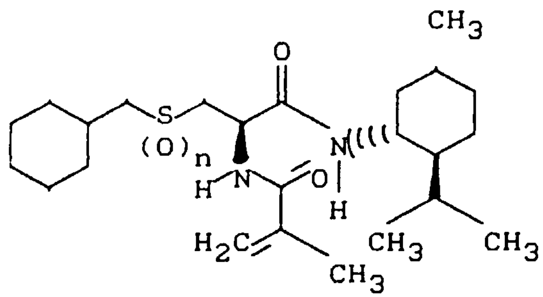

$\alpha$ = Enantioselektivität

$k_1'$ = Kapazitätsverhältnis

| Racemat | n=0 (Sulfid) | n= 1 (Sulfoxid) | Elutionsmittel |
|---|---|---|---|
| Nr. 1 | $\alpha$= 1,00 (keine Trennung) | $\alpha$=1,79 $k_1'$=1,09 | n-Heptan:THF = 1:1 |
| Nr. 2 | $\alpha$= 1,00 (keine Trennung) | $\alpha$=5,82 $k_1'$=0,39 | n-Heptan:THF = 1:4 |
| Nr. 3 | $\alpha$= 1,25 $k_1'$= 0,44 | $\alpha$=3,30 $k_1'$=0,62 | n-Heptan:THF = 1:4 |
| Nr. 5 | $\alpha$= 1,10 $k_1'$= 0,71 | $\alpha$=1,18 $k_1'$=1,67 | n-Heptan: Isopropanol= 10:1 |
| Nr. 6 | $\alpha$= 1,00 (keine Trennung) | $\alpha$=1,24 $k_1'$= 0,98 | n-Heptan: Isopropanol = 10:1 |
| Nr. 7 | $\alpha$= 1,00 (keine Trennung) | $\alpha$=1,48 $k_1'$= 0,85 (+) vor (-) | n-Heptan: THF = 1:1 |

## Patentansprüche

**Patentansprüche für folgende Vertragsstaaten : AT, BE, CH, DE, DK, FR, GB, IE, IT, LI, LU, MC, NL, PT, SE**

**1.** Optisch aktive S-Oxide enthaltende Aminosäurederivate der Formel (I)

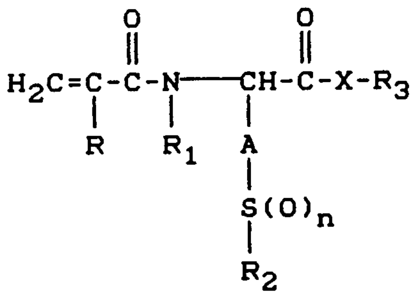

(I),

in der

n den Wert 1 oder 2 hat,

R für Wasserstoff, Methyl oder Fluor steht,

$R_1$ für Wasserstoff oder $C_1$-$C_4$-Alkyl steht oder zusammen mit $R_2$ eine Methylengruppe oder eine Dimethylengruppe bildet, die ein- oder zweifach mit $C_1$-$C_4$-Alkyl substituiert sein können,

$R_2$ für einen geradkettigen, verzweigten oder cyclischen Alkylrest mit bis zu 10 C-Atomen, $C_6$-$C_{14}$-Aryl,

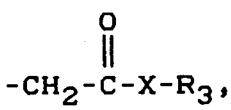

$C_2$-$C_{10}$-Acyl,
gegebenenfalls substituiertes Benzoyl- oder Benzyl steht oder zusammen mit $R_1$ eine dort beschriebene Brücke bildet,

$R_3$ für einen geradkettigen, verzweigten oder cyclischen Alkylrest mit bis zu 20 C-Atomen steht, der gegebenenfalls ein- bis dreifach substituiert ist durch Halogen, Alkoxy mit 1 bis 4 C-Atomen, Aralkoxy mit 7 bis 16 C-Atomen oder Aryl mit 6 bis 10 C-Atomen,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der ein- oder zweifach $C_1$-$C_4$-alkyl- oder $C_1$-$C_6$-alkoxycarbonylsubstituiert sein kann,

A für eine gegebenenfalls ein- oder zweifach $C_1$-$C_4$-alkylsubstituierte Methylen- oder Dimethylen-Gruppe steht.

**2.** Verbindungen der allgemeinen Formel (I) gemäß Anspruch 1, in welcher

n den Wert 1 oder 2 hat,

R für Wasserstoff, Methyl oder Fluor steht,

$R_1$ Wasserstoff oder Methyl bedeutet oder zusammen mit $R_2$ eine Methylengruppe, die ein- oder zweifach Methyl- oder einfach tertiär-Butylsubstituiert sein kann, oder eine Dimethylengruppe bildet,

$R_2$ für Alkyl mit bis zu 8 C-Atomen, Phenyl, Benzyl, Benzoyl und

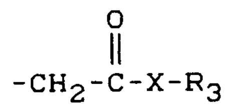

steht
oder zusammen mit $R_1$ eine dort beschriebene Brücke bildet,

$R_3$ für einen $C_{10}$-Terpenylrest, einen Adamantylrest, einen Decahydronaphthylrest, einen 1-Phenylethyl-Rest, einen $C_5$-$C_{12}$-Cycloalkylrest wie Cyclopentyl-, Cyclohexyl- oder Cycloheptyl- oder einen verzweigten $C_3$-$C_{12}$-Alkylrest wie Isopropyl, 3-Pentyl oder 5-Heptyl steht,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der gegebenenfalls ein-oder zweifach $C_1$-$C_4$-alkyl-oder $C_1$-$C_6$-alkoxycarbonylsubstituiert sein kann,

A für eine Methylen-, Dimethylmethylen- oder eine Dimethyleneinheit steht.

**3.** Verbindungen der allgemeinen Formel (I) gemäß Anspruch 1, dadurch gekennzeichnet, daß sie die Aminosäuresequenz der optisch aktiven schwefelhaltigen Aminosäuren Cystein, Homocystein, Penicillamin enthalten, deren SH-Funktion zum ersten alkyliert, aryliert, alkoxycarbonylmethyliert oder über eine Alkylenbrücke mit der Aminogruppe verbunden ist und zweitens zum Sulfoxid oder Sulfon oxidiert ist.

**4.** Verfahren zur Herstellung von Verbindungen der allgemeinen Formel (I)

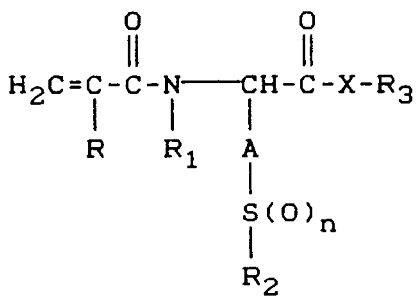

(I),

in der n, R, $R_1$, $R_2$, $R_3$, A und X die in Anspruch 1 angegebene Bedeutung haben, dadurch gekennzeichnet, daß man

A. optisch aktive S-Oxide enthaltende Aminosäurederivate der Formel (II)

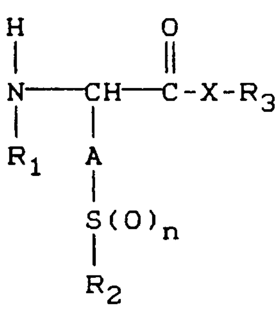

(II),

in der
n, $R_1$, $R_2$, $R_3$, A und X die unter Formel (I) angegebene Bedeutung haben und wo für n=1 sowohl die bezüglich des asymmetrischen Schwefelzentrums reinen Diastereomeren als auch die Gemische gemeint sind, oder

deren Säureadditionsprodukte mit Acryloyl-Derivaten der Formel (III)

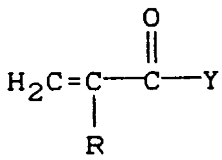

(III),

in der

R die unter Formel (I) angegebene Bedeutung hat und

Y für Fluor, Chlor oder Brom steht oder für den Rest

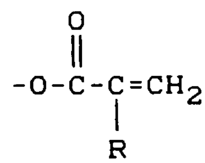

(Anhydrid) steht,

gegebenenfalls in Gegenwart eines Säurebindemittels in inerten organischen Lösungsmitteln umsetzt oder

B. optisch aktive schwefelhaltige Aminosäurederivate der Formel (IV)

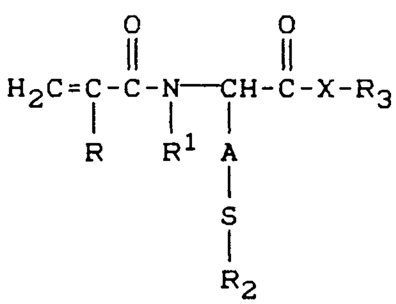

(IV),

in der
R, $R_1$, $R_2$, $R_3$, A und X die unter Formel (I) angegebene Bedeutung haben,
mit Oxidationsmitteln wie Wasserstoffperoxid, Natriummetaperiodat, 3-Chlorperbenzoesäure, Magnesiummonoperoxyphthalsäure, Tripelsalz der Caro'schen Säure ($KHSO_5xKHSO_4xK_2SO_4$) und anderen Sauerstoffüberträgern, die geeignet sind, Thioether in Sulfoxide und Sulfone zu überführen, zu den erfindungsgemäßen optisch aktiven S-Oxiden der Formel (I) oxidiert.

5. Optisch aktive Polymere, enthaltend mindestens 40 Mol-% Struktureinheiten der Formel (V)

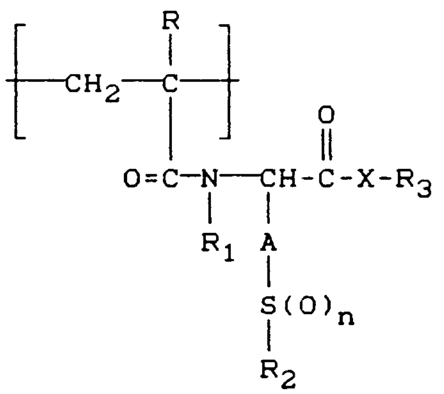

(V)

in welcher n, R, $R_1$, $R_2$, $R_3$, A und X die unter Formel (I) angegebene Bedeutung haben.

6. Optisch aktive Polymere gemäß Anspruch 5, dadurch gekennzeichnet, daß sie in Form von vernetzten unlöslichen aber quellbaren Perlpolymerisaten oder in an feinteilige anorganische Trägermaterialien gebundener Form vorliegen.

7. Verfahren zur Herstellung von vernetzten Perlpolymerisaten gemäß Anspruch 6, dadurch gekennzeichnet, daß man die S-Oxide enthaltenden N-Acryloyl-aminosäure-Derivate der allgemeinen Formel (I) gemäß Anspruch 1 mit 0,5 bis 50 Mol-% eines Vernetzers durch Suspensionspolymerisation, gegebenenfalls in Anwesenheit von Radikalbildnern und inerten organischen Lösungsmitteln herstellt.

8. Verfahren zur Herstellung von an anorganischen Trägermaterialien gebundenen optisch aktiven Polymere gemäß Anspruch 6, dadurch gekennzeichnet, daß man Verbindungen der allgemeinen Formel (I) gemäß Anspruch 1 in Gegenwart von gegebenenfalls (Meth)acryloyl-substituierten Kieselgel-Diolphasen oder von Kieselgel-Vinylphasen gegebenenfalls in Anwesenheit von Radikalbildnern und gegebenenfalls in Anwesenheit von inerten organischen Lösungsmitteln polymerisiert.

9. Verwendung von Polymeren gemäß Anspruch 6, zur Trennung von racemischen Gemischen in die optischen Antipoden.

**Patentansprüche für folgende Vertragsstaaten : ES, GR**

1. Verfahren zur Herstellung von Verbindungen der allgemeinen Formel (I)

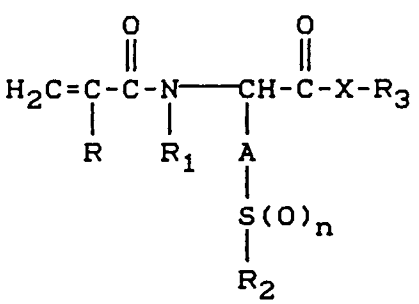

(I),

in der

n den Wert 1 oder 2 hat,

R für Wasserstoff, Methyl oder Fluor steht,

$R_1$ für Wasserstoff oder $C_1$-$C_4$-Alkyl steht oder zusammen mit $R_2$ eine Methylengruppe oder eine Dimethylengruppe bildet, die ein- oder zweifach mit $C_1$-$C_4$-Alkyl substituiert sein können,

$R_2$ für einen geradkettigen, verzweigten oder cyclischen Alkylrest mit bis zu 10 C-Atomen, $C_6$-$C_{14}$-Aryl,

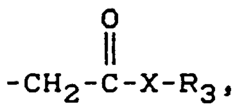

$C_2$-$C_{10}$-Acyl,
gegebenenfalls substituiertes Benzoyl- oder Benzyl steht oder zusammen mit $R_1$ eine dort beschriebene Brücke bildet,

$R_3$ für einen geradkettigen, verzweigten oder cyclischen Alkylrest mit bis zu 20 C-Atomen steht, der gegebenenfalls ein- bis dreifach substituiert ist durch Halogen, Alkoxy mit 1 bis 4 C-Atomen, Aralkoxy mit 7 bis 16 C-Atomen oder Aryl mit 6 bis 10 C-Atomen,

X Sauerstoff oder eine $NR_4$-Gruppe bedeutet, in der $R_4$ für Wasserstoff oder $C_1$-$C_4$-Alkyl steht oder zusammen mit $R_3$ einen stickstoffhaltigen 5- bis 7-gliedrigen Ring bildet, der ein- oder zweifach $C_1$-$C_4$-alkyl- oder $C_1$-$C_6$-alkoxy-carbonylsubstituiert sein kann,

A für eine gegebenenfalls ein- oder zweifach $C_1$-$C_4$-alkylsubstituierte Methylen- oder Dimethylen-Gruppe steht,

dadurch gekennzeichnet, daß man

A. optisch aktive S-Oxide enthaltende Aminosäurederivate der Formel (II)

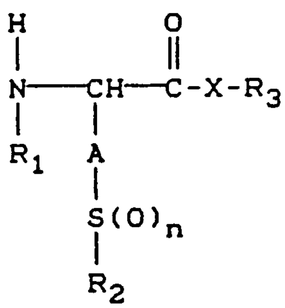

(II),

in der
n, $R_1$, $R_2$, $R_3$, A und X die unter Formel (I) angegebene Bedeutung haben und wo für n=1 sowohl die bezüglich des asymmetrischen Schwefelzentrums reinen Diastereomeren als auch die Gemische gemeint sind, oder deren Säureadditionsprodukte mit Acryloyl-Derivaten der Formel (III)

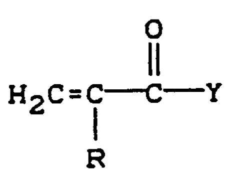

(III),

in der

R die unter Formel (I) angegebene Bedeutung hat und

Y für Fluor, Chlor oder Brom steht oder für den Rest

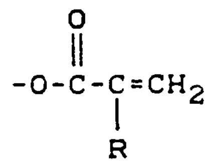

(Anhydrid) steht,

gegebenenfalls in Gegenwart eines Säurebindemittels in inerten organischen Lösungsmitteln umsetzt oder

B. optisch aktive schwefelhaltige Aminosäurederivate der Formel (IV)

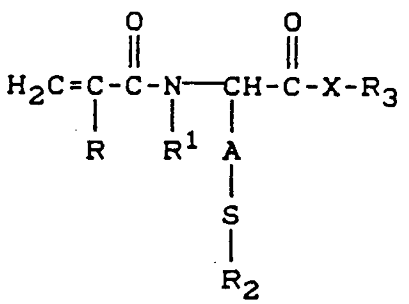

(IV),

in der
R, $R_1$, $R_2$, $R_3$, A und X die unter Formel (I) angegebene Bedeutung haben,
mit Oxidationsmitteln wie Wasserstoffperoxid, Natriummeteperiodat, 3-Chlorperbenzoesäure, Magnesiummonoperoxyphthalsäure, Tripelsalz der Caro'schen Säure ($KHSO_5xKHSO_4xK_2SO_4$) und anderen Sauerstoffüberträgern, die geeignet sind, Thioether in Sulfoxide und Sulfone zu überführen, zu den erfindungsgemäßen optisch aktiven S-Oxiden der Formel (I) oxidiert.

2. Verfahren zur Herstellung von optisch aktiven Polymeren, enthaltend mindestens 40 Mol-% Struktureinheiten der Formel V

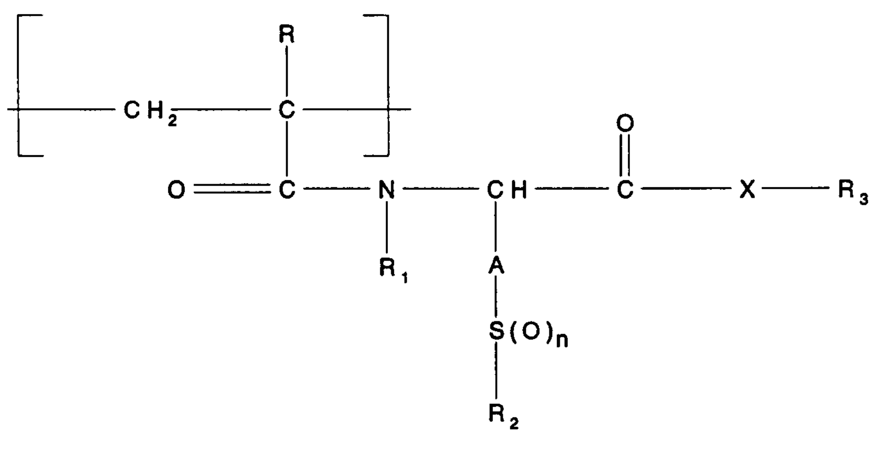

(V)

in welcher n, R, $R_1$, $R_2$, $R_3$, A und X die in Anspruch 1 unter Formel I angegebene Bedeutung haben, die in Form von vernetzten, unlöslichen aber quellbaren Perlpolymerisaten vorliegen, dadurch gekennzeichnet, daß man die S-Oxide enthaltenden N-Acryloyl-aminosäure-Derivate der allgemeinen Formel I gemäß Anspruch 1 mit 0,5 bis 50 Mol-% eines Vernetzers durch Suspensionspolymerisation, gegebenenfalls in Anwesenheit von Radikalbildnern und inerten organischen Lösungsmitteln herstellt.

3. Verfahren zur Herstellung von an anroganischen Trägermaterialien gebundenen optisch aktiven Polymeren enthaltend mindestens 40 Mol-% der Formel (V) gemäß Anspruch 2, in welcher n, R, $R_1$, $R_2$, $R_3$, A und X die in Anspruch 1 unter Formel I angegebene Bedeutung haben, dadurch gekennzeichnet, daß man Verbindungen der allgemeinen Formel I gemäß Anspruch 1 in Gegenwart von gegebenenfalls (Meth)acryloyl-substituierten Kieselgel-Diolphasen oder von Kieselgel-Vinylphasen gegebenenfalls in Anwesenheit von Radikalbildnern und gegebenenfalls in Anwesenheit von inerten organischen Lösungsmitteln polymerisiert.

4. Verwendung von Polymeren gemäß Ansprüchen 2 und 3 zur Trennung von racemischen Gemischen in die optischen Antipoden.

**Claims**

**Claims for the following Contracting States : AT, BE, CH, DE, DK, FR, GB, IE, IT, LI, LU, MC, NL, PT, SE**

1. Optically active S-oxide amino acid derivatives of the formula (I)

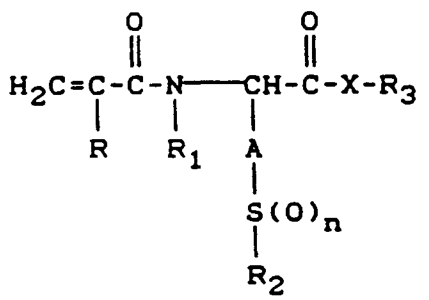

(I)

in which

n has the value 1 or 2,

R represents hydrogen, methyl or fluorine,

$R_1$ represents hydrogen or $C_1$-$C_4$-alkyl or together with $R_2$ forms a methylene group or a dimethylene group, each of which can be mono- or disubstituted by $C_1$-$C_4$-alkyl,

$R_2$ represents a straight-chain, branched or cyclic alkyl radical having up to 10 C atoms, $C_6$-$C_{14}$-aryl,

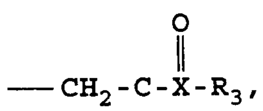

$C_2$-$C_{10}$-acyl, substituted or unsubstituted benzoyl or benzyl or together with $R_1$ forms a bridge described there,

$R_3$ represents a straight-chain, branched or cyclic alkyl radical having up to 20 C atoms which is unsubstituted or mono- to trisubstituted by halogen, alkoxy having 1 to 4 C atoms, aralkoxy having 7 to 16 C atoms or aryl having 6 to 10 C atoms,

X denotes oxygen or an $NR_4$ group in which $R_4$ represents hydrogen or $C_1$-$C_4$-alkyl or together with $R_3$ forms a nitrogen-containing 5- to 7-membered ring which may be mono- or disubstituted by $C_1$-$C_4$-alkyl- or $C_1$-$C_6$-alkoxycarbonyl,

A represents a methylene or dimethylene group which is unsubstituted or mono- or disubstituted by $C_1$-$C_4$-alkyl.

2. Compounds of the general formula (I) according to Claim 1, in which

n has the value 1 or 2,

R represents hydrogen, methyl or fluorine,

$R_1$ denotes hydrogen or methyl or together with $R_2$ forms a methylene group, which can be mono- or disubstituted by methyl or monosubstituted by tertiary-butyl, or a dimethylene group,

$R_2$ represents alkyl having up to 8 C atoms, phenyl, benzyl, benzoyl and,

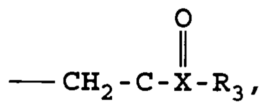

or
together with $R_1$ forms a bridge described there,

$R_3$ represents a $C_{10}$-terpenyl radical, an adamantyl radical, a decahydronaphthyl radical, a 1-phenylethyl radical, a $C_5$-$C_{12}$-cycloalkyl radical, such as cyclopentyl, cyclohexyl or cycloheptyl, or a branched $C_3$-$C_{12}$-alkyl radical, such as isopropyl, 3-pentyl or 5-heptyl,

X denotes oxygen or an $NR_4$ group in which $R_4$ represents hydrogen or $C_1$-$C_4$-alkyl or together with $R_3$ forms a nitrogen-containing 5- to 7-membered ring which may be unsubstituted or mono- or disubstituted by $C_1$-$C_4$-alkyl- or $C_1$-$C_6$-alkoxycarbonyl,

A represents a methylene, dimethylmethylene or dimethylene unit.

3. Compounds of the general formula (I) according to Claim 1, characterised in that they contain the amino acid sequence of the optically active sulphur-containing amino acids cysteine, homocysteine, penicillamine, whose SH function has first been alkylated, arylated, alkoxycarbonylmethylated or linked to the amino group via an alkylene bridge and has secondly been oxidised to the sulphoxide or sulphone.

4. Process for the preparation of compounds of the general formula (I)

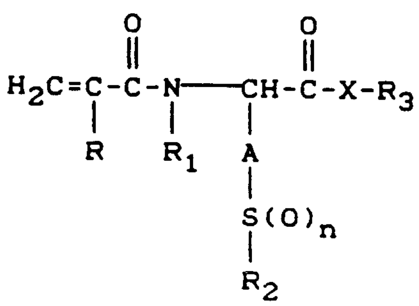

(I)

in which n, R, $R_1$, $R_2$, $R_3$, A and X have the meaning given in Claim 1, characterised in that

A. optically active S-oxide amino acid derivatives of the formula (II)

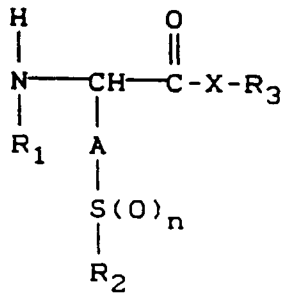

(II)

in which
n, $R_1$, $R_2$, $R_3$, A and X have the meaning given under formula (I) and which, in the case of n=1, are understood to mean not only the pure diastereomers with respect to the asymmetric sulphur centre but also the mixtures, or acid addition products thereof with acryloyl derivatives of the formula (III)

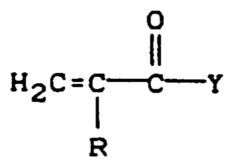

(III)

in which

R has the meaning given under formula (I) and

Y represents fluorine, chlorine or bromine or the radical

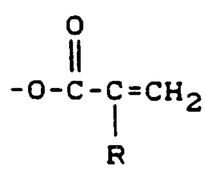

(anhydride),

are reacted, if appropriate in the presence of an acid-binding agent, in inert organic solvents, or

B. optically active sulphur-containing amino acid derivatives of the formula (IV)

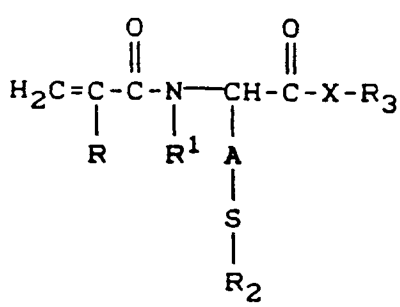

(IV)

in which

R, $R_1$, $R_2$, $R_3$, A and X have the meaning given under formula (I), are oxidised to the optically active S-oxides according to the invention of the formula (I) with oxidising agents, such as hydrogen peroxide, sodium metaperiodate, 3-chloroperbenzoic acid, magnesium monoperoxyphthalic acid, the triple salt of Caro's acid ($KHSO_5xKHSO_4xK_2SO_4$) and other oxygen carriers which are suitable for converting thioethers into sulphoxides and sulphones.

**5.** Optically active polymers containing at least 40 mol% of structural units of the formula (V)

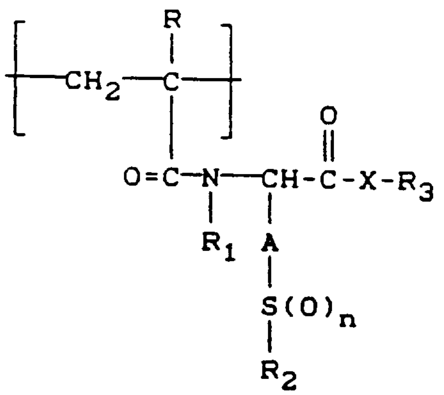

(V)

in which n, R, $R_1$, $R_2$, $R_3$, A and X have the meaning given under formula (I).

**6.** Optically active polymers according to Claim 5, characterised in that they are present in the form of crosslinked insoluble but swellable bead polymers or in a form bound to finely divided inorganic support materials.

**7.** Process for the preparation of crosslinked bead polymers according to Claim 6, characterised in that the S-oxide N-acryloyl amino acid derivatives of the general formula (I) according to Claim 1 are prepared in the presence of 0.5 to 50 mol% of a crosslinking agent by suspension polymerisation, if appropriate in the presence of free radical formers and inert organic solvents.

**8.** Process for the preparation of optically active polymers bound to inorganic support materials according to Claim 6, characterised in that compounds of the general formula (I) according to Claim 1 are polymerised in the presence of unsubstituted or (meth)acryloyl-substituted silica gel/diol phases or silica gel/vinyl phases, if appropriate in the presence of free radical formers and, if appropriate, in the presence of inert organic solvents.

**9.** Use of the polymers according to Claim 6 for the resolution of racemic mixtures to give the optical antipodes.

**Claims for the following Contracting States : ES, GR**

**1.** Process for the preparation of compounds of the general formula (I)

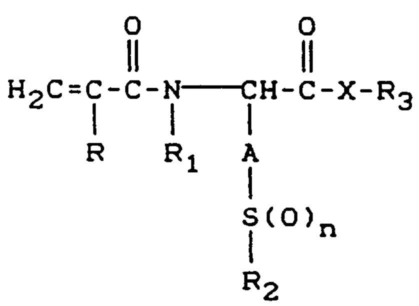

(I)

in which

n has the value 1 or 2,

R represents hydrogen, methyl or fluorine,

$R_1$ represents hydrogen or $C_1$-$C_4$-alkyl or together with $R_2$ forms a methylene group or a dimethylene group, each of which can be mono- or disubstituted by $C_1$-$C_4$-alkyl,

$R_2$ represents a straight-chain, branched or cyclic alkyl radical having up to 10 C atoms, $C_6$-$C_{14}$-aryl,

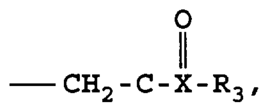

$C_2$-$C_{10}$-acyl, substituted or unsubstituted benzoyl or benzyl or together with $R_1$ forms a bridge described there,

$R_3$ represents a straight-chain, branched or cyclic alkyl radical having up to 20 C atoms which is unsubstituted or mono- to trisubstituted by halogen, alkoxy having 1 to 4 C atoms, aralkoxy having 7 to 16 C atoms or aryl having 6 to 10 C atoms,

X denotes oxygen or an $NR_4$ group in which $R_4$ represents hydrogen or $C_1$-$C_4$-alkyl or together with $R_3$ forms a nitrogen-containing 5- to 7-membered ring which may be mono- or disubstituted by $C_1$-$C_4$-alkyl- or $C_1$-$C_6$-alkoxycarbonyl,

A represents a methylene or dimethylene group which is unsubstituted or mono- or disubstituted by $C_1$-$C_4$-alkyl,

characterised in that

A. optically active S-oxide amino acid derivatives of the formula (II)

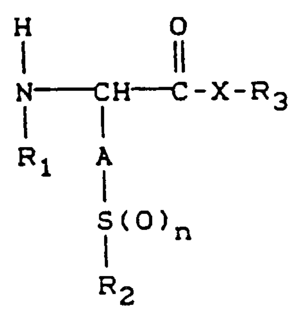

(II)

in which
n, $R_1$, $R_2$, $R_3$, A and X have the meaning given under formula (I) and which, in the case of n=1, are understood to mean not only the pure diastereomers with respect to the asymmetric sulphur centre but also the mixtures, or acid addition products thereof with acryloyl derivatives of the formula (III)

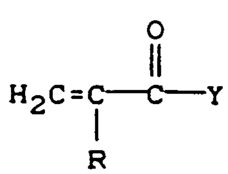

(III)

in which

R has the meaning given under formula (I) and

Y represents fluorine, chlorine or bromine or the radical

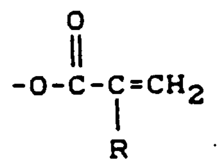

(anhydride),

are reacted, if appropriate in the presence of an acid-binding agent, in inert organic solvents, or

B. optically active sulphur-containing amino acid derivatives of the formula (IV)

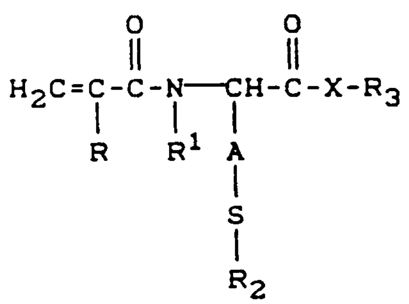

(IV)

in which
R, $R_1$, $R_2$, $R_3$, A and X have the meaning given under formula (I),
are oxidised to the optically active S-oxides according to the invention of the formula (I) with oxidising agents, such as hydrogen peroxide, sodium metaperiodate, 3-chloroperbenzoic acid, magnesium monoperoxyphthalic acid, the triple salt of Caro's acid ($KHSO_5xKHSO_4xK_2SO_4$) and other oxygen carriers which are suitable for converting thioethers into sulphoxides and sulphones.

2. Process for the preparation of optically active polymers containing at least 40 mol% of structural units of the formula V

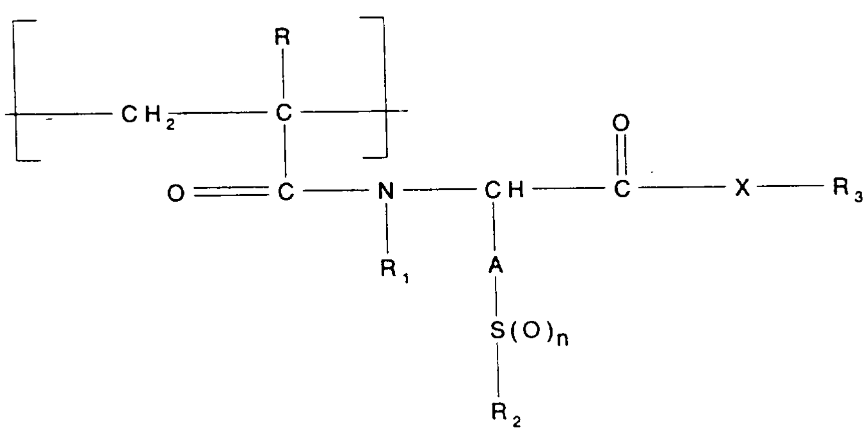

(V)

in which n, R, $R_1$, $R_2$, $R_3$, A and X have the meaning given in Claim 1 under formula I, which polymers are present in the form of crosslinked, insoluble but swellable bead polymers, characterised in that the S-oxide N-acryloyl amino acid derivatives of the general formula I according to Claim 1 are prepared in the presence of 0.5 to 50 mol% of a

crosslinking agent by suspension polymerisation, if appropriate in the presence of free radical formers and inert organic solvents.

3. Process for the preparation of optically active polymers which are bound to inorganic support materials and contain at least 40 mol% of the formula (V) according to Claim 2, in which n, R, $R_1$, $R_2$, $R_3$, A and X have the meaning given in Claim 1 under formula I, characterised in that compounds of the general formula I according to Claim 1 are polymerised in the presence of unsubstituted or (meth)acryloyl-substituted silica gel/diol phases or silica gel/vinyl phases, if appropriate in the presence of free radical formers and, if appropriate, in the presence of inert organic solvents.

4. Use of the polymers according to Claims 2 and 3 for the resolution of racemic mixtures to give the optical antipodes.

**Revendications**

**Revendications pour les Etats contractants suivants : AT, BE, CH, DE, DK, FR, GB, IE, IT, LI, LU, MC, NL, PT, SE**

1. Dérivés optiquement actifs d'aminoacides contenant des S-oxydes, de formule (I)

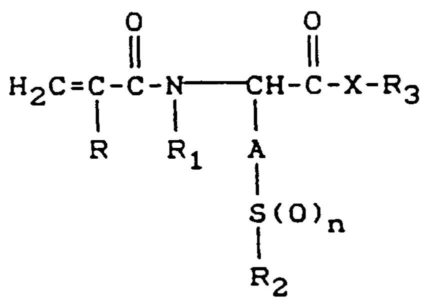

(I),

dans laquelle

- n a la valeur 1 ou 2,
- R est de l'hydrogène, un groupe méthyle ou du fluor,
- $R_1$ est de l'hydrogène ou un groupe alkyle en $C_1$ à $C_4$ ou forme conjointement avec $R_2$ un groupe méthylène ou un groupe diméthylène qui peuvent porter chacun un ou deux substituants alkyle en $C_1$ à $C_4$,
- $R_2$ est un reste alkyle linéaire, ramifié ou cyclique ayant jusqu'à 10 atomes de carbone, aryle en $C_6$ à $C_{14}$,

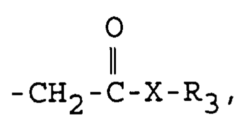

acyle en $C_2$ à $C_{10}$, un reste benzoyle ou benzyle facultativement substitué ou forme conjointement avec $R_1$ un pont tel que défini,
- $R_3$ est un reste alkyle linéaire, ramifié ou cyclique ayant jusqu'à 20 atomes de carbone, qui est substitué, le cas échéant, une à trois fois par un halogène, un radical alkoxy ayant 1 à 4 atomes de carbone, aralkoxy ayant 7 à 16 atomes de carbone ou aryle ayant 6 à 10 atomes de carbone,
- X représente de l'oxygène ou un groupe $NR_4$ dans lequel $R_4$ est de l'hydrogène ou un radical alkyle en $C_1$ à $C_4$, ou forme conjointement avec $R_3$ un noyau pentagonal à heptagonal contenant de l'azote, qui peut être substitué une ou deux fois par un radical (alkyle en $C_1$ à $C_4$)-carbonyle ou (alkoxy en $C_1$ à $C_6$)-carbonyle,
- A est un groupe méthylène ou diméthylène portant, le cas échéant, un ou deux substituants alkyle en $C_1$ à $C_4$.

2. Composés de formule générale (I) suivant la revendication 1, dans laquelle

- n a la valeur 1 ou 2,
- R est de l'hydrogène, un groupe méthyle ou du fluor,

$R_1$ est de l'hydrogène ou un groupe méthyle ou forme conjointement avec $R_2$ un groupe méthylène qui peut être substitué une ou deux fois par un radical méthyle ou une fois par un radical tertio-butyle, ou bien un groupe diméthylène,

$R_2$ est un groupe alkyle ayant jusqu'à 8 atomes de carbone, phényle, benzyle, benzoyle et

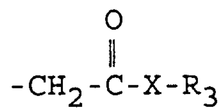

ou forme conjointement avec $R_1$ un pont tel que défini,

$R_3$ est un reste terpényle en $C_{10}$, un reste adamantyle, un reste décahydronaphtyle, un reste 1-phényléthyle, un reste cycloalkyle en $C_5$ à $C_{12}$ tel qu'un reste cyclopentyle, cyclohexyle ou cycloheptyle ou un reste alkyle en $C_3$ à $C_{12}$ ramifié tel qu'isopropyle, 3-pentyle ou 5-heptyle,

X représente de l'oxygène ou un groupe $NR_4$ dans lequel $R_4$ est de l'hydrogène ou un radical alkyle en $C_1$ à $C_4$ ou forme conjointement avec $R_3$ un noyau pentagonal à heptagonal contenant de l'azote, qui peut être substitué, le cas échéant, une ou deux fois par un radical (alkyle en $C_1$ à $C_4$)-carbonyle ou (alkoxy en $C_1$ à $C_6$)-carbonyle,

A est un motif méthylène, diméthylméthylène ou diméthylène.

**3.** Composés de formule générale (I) suivant la revendication 1, caractérisés en ce qu'ils contiennent la séquence des aminoacides optiquement actifs, contenant du soufre, cystéine, homocystéine, pénicillamine dont la fonction SH est tout d'abord alkylée, arylée, alkoxycarbonylméthylée ou reliée par l'intermédiaire d'un pont alkylène au groupe amino, et oxydée en second lieu en sulfoxyde ou en sulfone.

**4.** Procédé de production de composés de formule générale (I)

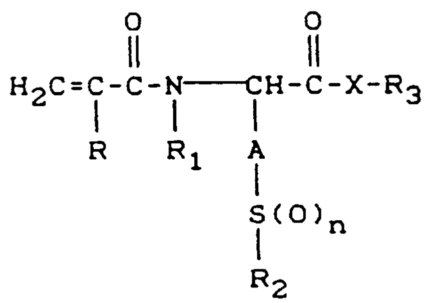

(I),

dans laquelle <u>n</u>, R, $R_1$, $R_2$, $R_3$, A et X ont la définition indiquée dans la revendication 1, caractérisé en ce que

A. on fait réagir des dérivés optiquement actifs d'aminoacides contenant des S-oxydes, de formule (II)

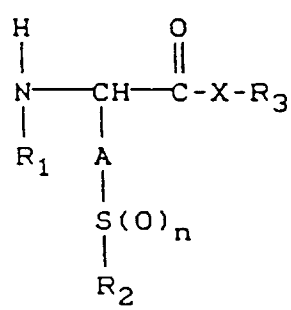

(II),

dans laquelle

<u>n</u>, $R_1$, $R_2$, $R_3$, A et X ont la définition indiquée pour la formule (I) et où, pour n=1, on considère aussi bien les diastéréoisomères purs par rapport au centre d'asymétrie du soufre que les mélanges, ou leurs produits

d'addition d'acides avec des dérivés d'acryloyle de formule (III)

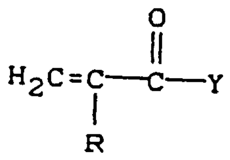

(III),

dans laquelle

R a la définition indiquée pour la formule (I) et
Y représente du fluor, du chlore ou du brome ou le reste

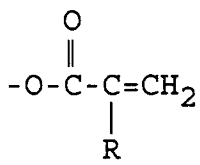

(anhydride),

le cas échéant en présence d'un accepteur d'acide dans des solvants organiques inertes, ou bien B. on oxyde en les S-oxydes optiquement actifs de formule (I) conformes à l'invention des dérivés optiquement actifs d'aminoacides contenant du soufre de formule (IV)

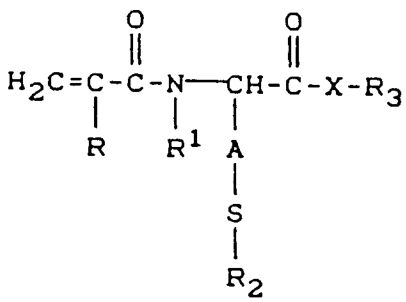

(IV),

dans laquelle
R, $R_1$, $R_2$, $R_3$, A et X ont la définition indiquée pour la formule (I),
avec des agents oxydants tels que le peroxyde d'hydrogène, le métaperiodate de sodium, l'acide 3-chloroperbenzoïque, l'acide magnésium-monoperoxyphtalique, le sel triple de l'acide de Caro ($KHSO_5xKHSO_4xK_2SO_4$) et d'autres véhiculeurs d'oxygène, qui conviennent pour transformer des thioéthers en sulfoxydes et sulfones.

5. Polymères optiquement actifs, contenant au moins 40 moles % de motifs structuraux de formule (V)

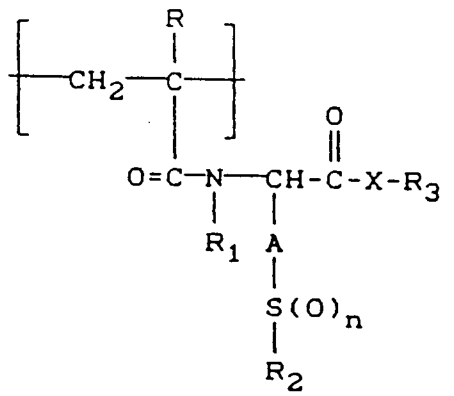

(V)

dans laquelle n, R, $R_1$, $R_2$, $R_3$, A et X ont la définition indiquée pour la formule (I).

6. Polymères optiquement actifs suivant la revendication 5, caractérisés en ce qu'ils se présentent sous forme de produits de polymérisation en perles réticulés, insolubles mais gonflants ou sous une forme liée à des matières inorganiques de support en fines particules.

7. Procédé de production de polymères en perles réticulés suivant la revendication 6, caractérisé en ce qu'on produit les dérivés de N-acryloylaminoacides contenant des S-oxydes de formule générale (I) suivant la revendication 1 avec 0,5 à 50 moles % d'un agent de réticulation par polymérisation en suspension, le cas échéant en présence de générateurs de radicaux et de solvants organiques inertes.

8. Procédé de production de polymères optiquement actifs liés à des matières inorganiques de support suivant la revendication 6, caractérisé en ce qu'on polymérise des composés de formule générale (I) suivant la revendication 1 en présence de phases de diols-gel de silice ou de phases vinyliques-gel de silice portant éventuellement un substituant (méth)acryloyle, éventuellement en présence de générateurs radicalaires et, le cas échéant en présence de solvants organiques inertes.

9. Utilisation de polymères suivant la revendication 6, pour la séparation de mélanges racémiques en les antipodes optiques.

**Revendications pour les Etats contractants suivants : ES, GR**

1. Procédé de production de composés de formule générale (I)

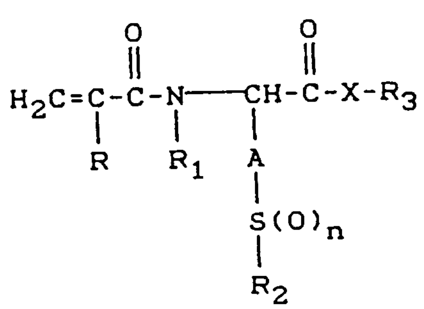

(I),

dans laquelle

n a la valeur 1 ou 2,
R est de l'hydrogène, un groupe méthyle ou du fluor,
$R_1$ est de l'hydrogène ou un groupe alkyle en $C_1$ à $C_4$ ou forme conjointement avec $R_2$ un groupe méthylène ou un groupe diméthylène qui peuvent porter chacun un ou deux substituants alkyle en $C_1$ à $C_4$,

$R_2$ est un reste alkyle linéaire, ramifié ou cyclique ayant jusqu'à 10 atomes de carbone, aryle en $C_6$ à $C_{14}$,

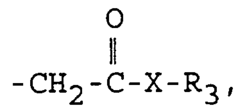

acyle en $C_2$ à $C_{10}$, un reste benzoyle ou benzyle facultativement substitué ou forme conjointement avec $R_1$ un pont tel que défini,

$R_3$ est un reste alkyle linéaire, ramifié ou cyclique ayant jusqu'à 20 atomes de carbone, qui est substitué, le cas échéant, une à trois fois par un halogène, un radical alkoxy ayant 1 à 4 atomes de carbone, aralkoxy ayant 7 à 16 atomes de carbone ou aryle ayant 6 à 10 atomes de carbone,

X représente de l'oxygène ou un groupe $NR_4$ dans lequel $R_4$ est de l'hydrogène ou un radical alkyle en $C_1$ à $C_4$, ou forme conjointement avec $R_3$ un noyau pentagonal à heptagonal contenant de l'azote, qui peut être substitué une ou deux fois par un radical (alkyle en $C_1$ à $C_4$)-carbonyle ou (alkoxy en $C_1$ à $C_6$)-carbonyle,

A est un groupe méthylène ou diméthylène portant, le cas échéant, un ou deux substituants alkyle en $C_1$ à $C_4$, caractérisé en ce que :

A. on fait réagir des dérivés optiquement actifs d'aminoacides contenant des S-oxydes, de formule (II)

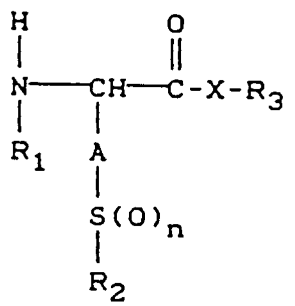

(II),

dans laquelle

n, $R_1$, $R_2$, $R_3$, A et X ont la définition indiquée pour la formule (I) et où, pour n=1, on considère aussi bien les diastéréoisomères purs par rapport au centre d'asymétrie du soufre que les mélanges, ou leurs produits d'addition d'acides avec des dérivés d'acryloyie de formule (III)

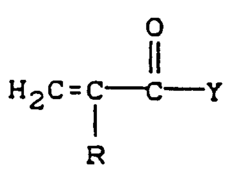

(III),

dans laquelle

R a la définition indiquée pour la formule (I) et

Y représente du fluor, du chlore ou du brome ou le reste

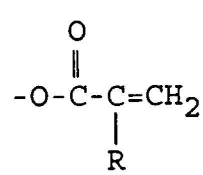

(anhydride),

le cas échéant en présence d'un accepteur d'acide dans des solvants organiques inertes, ou bien

B. on oxyde en les S-oxydes optiquement actifs de formule (I) conformes à l'invention des dérivés optiquement actifs d'aminoacides contenant du soufre de formule (IV)

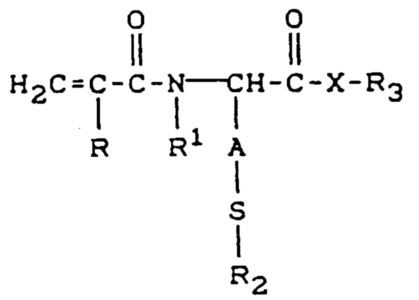

(IV),

dans laquelle

R, $R_1$, $R_2$, $R_3$, A et X ont la définition indiquée pour la formule (I),

avec des agents oxydants tels que le peroxyde d'hydrogène, le métaperiodate de sodium, l'acide 3-chloroperbenzoïque, l'acide magnésium-monoperoxyphtalique, le sel tripie de l'acide de Caro ($KHSO_5xKHSO_4xK_2SO_4$) et d'autres véhicuieurs d'oxygène, qui conviennent pour transformer des thioéthers en sulfoxydes et sulfones.

**2.** Procédé de production de polymères optiquement actifs contenant au moins 40 moles % de motifs structuraux de formule V

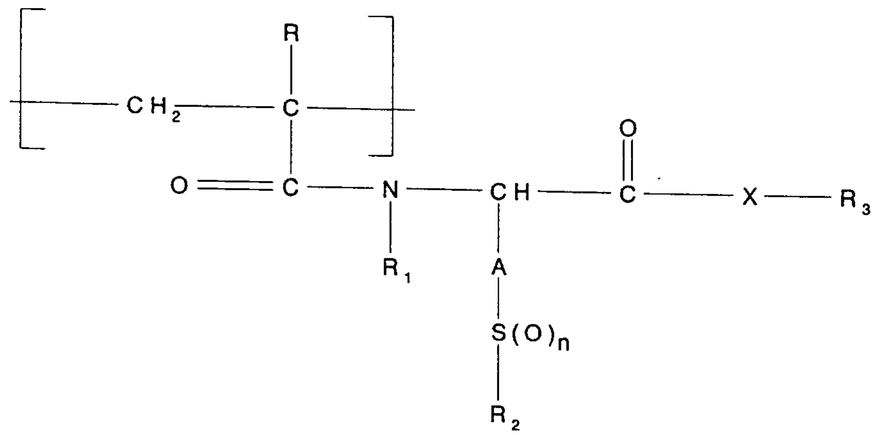

(V)

dans laquelle n, R, $R_1$, $R_2$, $R_3$, A et X ont la définition indiquée dans la revendication 1 pour la formule I, qui se présentent sous forme de produits de polymérisation en perles réticulés, non solubles mais gonflants, caractérisé en ce qu'on produit les dérivés de N-acryloylaminoacides contenant des S-oxydes de formule générale (I) suivant la revendication 1 avec 0,5 à 50 moles % d'un agent de réticulation par polymérisation en suspension, le cas échéant en présence de générateurs de radicaux et de solvants organiques inertes.

**3.** Procédé de production de polymères optiquement actifs liés à des matières inorganiques de support, contenant au moins 40 moles % de motifs structuraux de formule (V) suivant la revendication 2, dans laquelle n, R, $R_1$, $R_2$, $R_3$, A et X ont la définition indiquée pour la formule (I) dans la revendication 1, caractérisé en ce qu'on polymérise des composés de formule générale (I) suivant la revendication 1 en présence de phases de diols-gel de silice ou de phases vinyliques-gel de silice portant éventuellement un substituant (méth)acryloyle, éventuellement en présence de générateurs radicalaires et, le cas échéant en présence de solvants organiques inertes.

**4.** Utilisation de polymères suivant les revendications 2 et 3, pour séparer des mélanges racémiques en les antipodes optiques.